# FASCICULE DE BREVET EUROPEEN

(11) **EP 2 276 832 B1**
(45) Date de publication et mention de la délivrance du brevet: **21.12.2016**
(21) Numéro de dépôt: 09745821.0
(22) Date de dépôt: 15.05.2009
(51) Int. Cl.: C12N 5/0784, A61K 35/12, A61K 39/00

(54) **LIGNÉE DE CELLULES DENDRITIQUES PLASMACYTOÏDES UTILISÉE EN THÉRAPIE CELLULAIRE ACTIVE OU ADOPTIVE**
PLASMAZYTOIDE DENDRITISCHE ZELLLINIE IN DER AKTIVEN ODER ADOPTIVEN ZELLTHERAPIE
PLASMACYTOID DENDRITIC CELL LINE USED IN ACTIVE OR ADOPTIVE CELL THERAPY

(30) Priorité: 16.05.2008 FR 0802659
(43) Date de publication de la demande: 26.01.2011
(73) Titulaire: Etablissement Français du Sang, 93210 La Plaine Saint Denis (FR)
(72) Inventeur: PLUMAS, Joel, F-38000 Grenoble (FR); ASPORD, Caroline, 38000 Grenoble (FR); CHAPEROT-DUBONNET, Laurence, F-73800 Françin (FR)
(74) Mandataire: Lavoix
(86) Numéro de dépôt international: PCT/EP2009/055909
(87) Numéro de publication internationale: WO 2009/138489

(56) Documents cités:
- FR-A- 2 848 565
- US-A1- 2004 265 998
- YEE C. ET AL.: "Adoptive T cell therapy using antigen-specific CD8+ T cell clones for the treatment of patients with metastatic melanoma: In vivo persistence, migration, and antitumor effect of transferred T cells." PROCEEDINGS OF THE NATIONAL ACADEMY OF SCIENCES OF THE UNITED STATES OF AMERICA, vol. 99, no. 25, 10 décembre 2002 (2002-12-10), pages 16168-16173, XP002505178 ISSN: 0027-8424
- S.J.A.M. SANTEGOETS ET AL.: "In vitro priming of tumor-specific cytotoxic T lymphocytes using allogeneic dendritic cells derived from the human MUTZ-3 cell line" CANCER IMMUNOLOGY, IMMUNOTHERAPY, SPRINGER, BERLIN, DE, vol. 55, no. 12, 9 février 2006 (2006-02-09), pages 1480-1490, XP019422502 ISSN: 1432-0851 cité dans la demande
- CHAPEROT L. ET AL: "Leukemic plasmacytoid dendritic cells share phenotypic and functional features with their normal counterparts." EUROPEAN JOURNAL OF IMMUNOLOGY, vol. 34, no. 2, février 2004 (2004-02), pages 418-426, XP002505517 ISSN: 0014-2980
- PLUMAS J. ET AL.: "Plasmacytoid dendritic cells capture and cross-present viral antigens from influenza-virus infected cells" BULLETIN DU CANCER, EDITIONS SCIENTIFIQUES ELSEVIER, PARIS, FR, vol. 95, no. sp. iss. Si, 1 mars 2008 (2008-03-01), page S32, XP009109113 ISSN: 0007-4551
- CHAPEROT L. ET AL.: "Identification of a leukemic counterpart of the plasmacytoid dendritic cells" BLOOD, AMERICAN SOCIETY OF HEMATOLOGY, US, vol. 97, no. 10, 15 mai 2001 (2001-05-15), pages 3210-3217, XP002243855 ISSN: 0006-4971

### Listage de Séquences

<110> Etablissement Français du Sang
<120> Lignée de cellules dendritiques plasmacitoides utilisées en thérapie cellulaire active ou adoptive
<130> BR057107
<160> 13
<170> PatentIn version 3.5
<210> 1
   <211> 10
   <212> PRT
   <213> Homo sapiens
<400> 1
   Glu Leu Ala Gly Ile Gly Ile Leu Thr Val
   1               5                   10
<210> 2
   <211> 9
   <212> PRT
   <213> Homo sapiens
<400> 2
   Ile Met Asp Gln Val Pro Phe Ser Val
   1               5
<210> 3
   <211> 9
   <212> PRT
   <213> Homo sapiens
<400> 3
   Tyr Met Asp Gly Thr Met Ser Gln Val
   1 5
<210> 4
   <211> 9
   <212> PRT
   <213> Homo sapiens
<400> 4
   Phe Leu Trp Gly Pro Arg Ala Leu Val
   1 5
<210> 5
   <211> 9
   <212> PRT
   <213> Homo sapiens
<400> 5
   Ser Leu Leu Met Trp Ile Thr Gln Cys
   1 5
<210> 6
   <211> 9
   <212> PRT
   <213> Human Immunodeficiency virus (HIV)
<220>
   <221> PEPTIDE
   <222> (1)..(9)
   <223> Peptide part of of the gag protein
<400> 6
   Ser Leu Tyr Asn Thr Val Ala Thr Leu
   1 5
<210> 7
   <211> 9
   <212> PRT
   <213> Human Immunodeficiency virus (HIV)
<220>
   <221> PEPTIDE
   <222> (1)..(9)
   <223> Peptide part of the pol protein
<400> 7
   Ile Leu Lys Glu Pro Val His Gly Val
   1 5
<210> 8
   <211> 9
   <212> PRT
   <213> Influenza virus
<400> 8
   Gly Ile Leu Gly Phe Val Phe Thr Leu
   1 5
<210> 9
   <211> 9
   <212> PRT
   <213> Cytomegalovirus (CMV)
<220>
   <221> PEPTIDE
   <222> (1)..(9)
   <223> peptide of part of the viral antigen pp65
<400> 9
   Asn Leu Val Pro Met Val Ala Thr Val
   1 5
<210> 10
   <211> 9
   <212> PRT
   <213> Epstein Barr Virus
<220>
   <221> PEPTIDE
   <222> (1)..(9)
   <223> peptide part of the viral antigen BMLF1
<400> 10
   Gly Leu Cys Thr Leu Val Ala Met Leu
   1 5
<210> 11
   <211> 9
   <212> PRT
   <213> Epstein Barr Virus
<220>
   <221> PEPTIDE
   <222> (1)..(9)
   <223> Peptide part of the viral antigen LMP2
<400> 11
   Cys Leu Gly Gly Leu Leu Thr Met Val
   1 5
<210> 12
   <211> 10
   <212> PRT
   <213> Epstein Barr Virus
<220>
   <221> PEPTIDE
   <222> (1)..(10)
   <223> Peptide part of the viral antigen EBNA-2
<400> 12
   Asp Thr Pro Leu Ile Pro Leu Thr Ile Phe
   1               5                   10
<210> 13
   <211> 9
   <212> PRT
   <213> Epstein Barr Virus
<220>
   <221> PEPTIDE
   <222> (1)..(9)
   <223> Peptide part of the viral antigen EBNA-3a
<400> 13
   Ser Val Arg Asp Arg Leu Ala Arg Leu
   1               5

## Description

La présente invention concerne une méthode d'induction et d'amplification d'effecteurs cytotoxiques mettant en oeuvre une lignée de cellules dendritiques plasmacytoïdes (pDC) en situation semi allogénique. L'invention se rapporte également à l'utilisation d'une lignée de pDC pour l'obtention d'un médicament destiné au traitement et ou à la prévention de maladies infectieuses ou de cancers. L'invention concerne également un vaccin comprenant des pDC et une méthode de traitement des cancers ou de maladies infectieuses.

### Les cellules dendritiques

Les cellules dendritiques sont des acteurs clés de la réponse immune : elles sont responsables de la capture des antigènes et de leur apprêtement en vue de leur présentation aux lymphocytes T. Il existe différents types de cellules dendritiques qui se distinguent par leur ontogénie et leurs capacités fonctionnelles : les cellules dendritiques myéloïdes (mDC) issues de précurseurs exprimant les molécules CD11c, CD13 et CD33, et les cellules dendritiques plasmacytoïdes (pDC), caractérisées par une très forte expression du récepteur à l'IL3 (CD123) et capables de maturer sous l'effet de l'IL3 et de CD40L, ou en présence d'un virus.

Les pDC, ont été identifiées précisément à partir d'amygdales en 1997 par G. Grouard et al. (Grouard G et al., J Exp Med., 1997;185:1101- 1111), elles ont été aussi décrites dans le sang (O'Doherty U. et al., Immunology, 1994;82:487-493 ; Robinson SP. et al., Eur J Immunol., 1999;29:2769-2778), dans les ganglions (Cella M. et al., Nat Med., 1999;5:919- 923) et le thymus (Res PC. et al., Blood, 1999;94:2647-2657 ; BendrissVermare N. et al., J Clin Invest., 2001;107:835-844). Ces cellules sont caractérisées par leur morphologie de type plasmacytoïde et leur phénotype.

Les pDC expriment les molécules CD4, HLA-DR et CD45RA, et sont dépourvues des marqueurs de type myéloïde CD11 c et CD13 (Cella M. et al., Nat Med., 1999;5:919-923), ou de marqueurs spécifiques de lignées tels que CD3, CD14 et CD19, bien qu'une expression de CD2, CD5 ou CD7 ait été parfois observée (Cella M. et al., Nat Med., 1999;5:919-923 ; Res PC. étal., Blood., 1999;94:2647-2657). Plus récemment, la lectine BDCA2 spécifiquement exprimée par les pDC a pu être identifiée; BDCA4 est retrouvée sur les pDC mais est aussi présente sur les DC dérivées de monocyte (Dzionek A. et al., J Immunol., 2000;165:6037-6046). Un argument en faveur de l'appartenance de ces cellules à la lignée lymphoïde est leur expression des ARNm codant pour les chaînes preTalpha (Res PC. et al., Blood., 1999;94:2647-2657), lambda like 14.1 et SpiB (Bendriss-Vermare N. et al., J Clin Invest., 2001;107:835-844). Ces cellules expriment très fortement le récepteur de l'IL-3 et faiblement le récepteur au GM-CSF (Cella M. et al., Nat Med., 1999;5:919-923 ; Rissoan MC. et al., Science., 1999;283:1183-1186), et ces deux cytokines favorisent la survie des pDC (Grouard G. et al., J Exp Med., 1997;185:1101-1111 ; Kohrgruber N. et al., J Immunol., 1999;163:3250-3259 ; Robinson SP. et al., Eur J Immunol., 1999;29:2769-2778) qui meurent sinon très rapidement *in vitro.* Les molécules de costimulation CD80 et CD86 sont absentes ou faiblement exprimées (Grouard G. et al., J Exp Med., 1997;185:1101-1111), et au stade immature, ces cellules ne sont pas capables d'activer des lymphocytes T (Kohrgruber N. et al., J Immunol., 1999;163:3250-3259). En revanche, en présence d'IL-3, de CD40L ou d'un virus, les pDC maturent, expriment fortement les molécules accessoires de la présentation des antigènes (CD40, CD80, CD86 et HLA-DR) et deviennent alors capables d'activer la prolifération de cellules T allogéniques (Kohrgruber N. et al., J Immunol., 1999;163:3250- 3259 ; Grabbe S. et al., Immunol Today., 2000;21:431-433 ; Kadowaki N. et al., J Exp Med., 2000;192:219-226). Selon le stimulus responsable de leur maturation (IL3 ou virus), les pDC vont polariser la réponse des lymphocytes T naïfs qu'elles activent, de manière plus ou moins stricte, vers un profil Th1 ou Th2 (Rissoan MC. et al., Science., 1999;283:1183-1186 ; Cella M. et al., Nat. Immunol., 2000;1:305-310 ; Kadowaki N. et al., J Exp Med., 2000;192:219- 226). Les pDC sont aussi décrites pour induire des réponses de type TCD4 et CD8 régulatrices (Gilliet & Liu, Hum. Immunol. 63, 2002 ; Wei et al., Cancer Res. 65(12), 2005 ; Gilliet & Liu, J. Exp. Med.,195(6), 2002).

### Les cellules dendritiques en contexte tumoral ou infectieux

Dans un contexte tumoral, les pDC présentent souvent un état immature et des fonctions tolérogènes (Perrot et al., The J. of Imm., 2007 ; Hartmann et al., Cancer Res., 63, 2003 ; Treilleux et al., Clinical Cancer Res ., 10, 2004). Il faut noter que l'infiltration de cancer du sein par des pDC est corrélée avec un pronostic de survie défavorable des patients, suggérant une contribution dans la progression dudit cancer (Treilleux *et al*., 2004), ou une inhibition de leurs fonctions dans le contexte du micro-environnement tumoral.

Dans le contexte d'infections virales, les pDC ont un rôle central dans l'initiation de la réponse antivirale, par leur sécrétion d'interféron alpha, et du fait de leur capacité à présenter les antigènes viraux, pour activer des lymphocytes T CD8+ cytotoxiques (Hoefel et al, 2008, Immunity 27 : 481-492 ; Di Pucchio, 2008, Nature Immunol. adv online publication).

### Immunothérapie active et immunothérapie allogénique

De nombreux essais cliniques démontrent la capacité des cellules dendritiques myéloïdes (mDC) autologues à stimuler des réponses immunes anti-tumorales (Thurner et al., 1999, J. Exp. Med. 190(11) : 1669-78 ; Palucka et al., 2005, J. Immunother. 28(2) : 158-68). Dans la plupart de ces essais, la génération des mDC se fait à partir de monocytes prélevés chez le patient, cultivés en présence de cytokines pendant plusieurs jours, et fait parfois intervenir une étape supplémentaire de maturation des cellules. La production de ces mDC doit donc être réalisée pour chaque patient individuellement, et les essais cliniques réalisés jusqu'à présent avec les mDC autologues ne permettent pas une efficacité clinique suffisante pour améliorer la survie globale des patients (Banchereau et al., 2005, Nat. Rev. Immunol. 5(4) : 296-306).

L'immunogénicité des vaccins cellulaires pourrait être améliorée dans un contexte allogénique, ainsi l'utilisation de mDC semi-allogéniques ou de lignées tumorales semi-allogéniques dans des protocoles d'immunothérapie est déjà documentée (Hus et al., 2005, Leukemia 19 : 1621-1627 ; Holtl et al., 2005, Cancer Immunol Immunother. 54 : 663-670). Les mDC peuvent être obtenues à partir de la lignée MUTZ3 une lignée de cellules myéloïdes CD34⁺ issue d'une leucémie. L'obtention de mDC à partir de cette lignée est complexe (stimulation par GM-CSF, IL-4 et TNF-a, puis maturation par adjonction de TNF-a, IL-6, IL-1b et PGE2). Ces mDC dérivées de MUTZ-3 sont décrites comme pouvant induire des cellules T cytotoxiques spécifiques d'antigènes tumoraux *in vitro* (Santegoets et al., 2006 Cancer Immunol. Immunother. 55 : 1480-1490, US 2004/0265998). Cependant le niveau d'induction de la réponse reste très faible, avec obtention de seulement 0,4% de lymphocytes T cytotoxique spécifiques de l'antigène parmi les lymphocytes T.

Par ailleurs, la lignée de cancer du sein KS transfectée avec la molécule de co-stimulation CD80 peut aussi induire des cellules T spécifiques d'antigènes tumoraux (Guckel, 2005, Cancer Immunol. Immunother., 54 : 129-140) et est déjà utilisée en essais cliniques de phase I/II. Mais la réponse induite est restreinte vis-à-vis d'antigènes exprimés par cette lignée (Her-2/Neu) et ne permet pas d'induire des réponses contre d'autres antigènes tumoraux ou viraux.

La présente invention apporte des solutions aux problèmes des stratégies d'immunothérapies actuelles, liés à la difficulté de mise en oeuvre des protocoles et de leur industrialisation, au manque d'efficacité thérapeutique et au fait que les stratégies sont développées pour une pathologie très ciblée donc ont un champ d'application très restreint.

En effet, la présente invention a pour objet une méthode d'induction et d'amplification d'effecteurs spécifiques mettant en oeuvre une lignée de pDC dans un contexte semi-allogénique, l'utilisation de ladite lignée pour la fabrication d'un médicament, notamment un vaccin ainsi qu'une méthode de prévention et/ou de traitement des cancers et/ou de maladies infectieuses.

### Description de l'invention

La présente invention concerne une méthode d'induction et d'amplification *in vitro* d'effecteurs spécifiques (c'est-à-dire des cellules de l'immunité capables de reconnaitre au moins un antigène spécifique) comprenant : (a) l'obtention d'une lignée de cellules dendritiques plasmacytoïdes (pDC) pulsées par incubation d'une lignée de pDC avec ledit au moins un antigène spécifique, (b) l'irradiation des cellules obtenues à l'étape (a), (c) la mise en contact de pDCs pulsées et irradiées obtenues à l'étape (b) avec des cellules mononuclées du sang périphérique (PBMC), et culture des pDC pulsées et irradiées et des PBMC partageant au moins un allèle du complexe majeur d'histocompatibilité (CMH).

Une amplification plus importante du nombre d'effecteurs spécifiques peut être obtenue par au moins une nouvelle mise en contact des cellules issues de la culture mentionnée ci-dessus avec des pDC pulsées et irradiées suivie d'une étape de culture.

Le terme « organisme », dans le cadre de la présente invention définit tout particulièrement l'homme ou la souris humanisée.

Le terme « lignée cellulaire » s'applique à des cellules de mammifère cultivées *in vitro.* Les cellules primaires de mammifères ne se multiplient pas en culture ou cessent de se multiplier en culture après un nombre limité de divisions. Les lignées cellulaires selon la présente invention sont capables de se multiplier indéfiniment, ce dont les cultures primaires ou secondaires de cellules de mammifère sont incapables. Ces propriétés des lignées de cellules dendritiques plasmacytoïdes humaines (pDC) selon l'invention permettent d'obtenir avantageusement des quantités importantes de cellules par multiplication ou prolifération de ces cellules *in vitro.*

Dans un mode de réalisation de l'invention, la lignée de pDC est obtenue à partir de cellules de leucémie à pDC. Le brevet européen EP 1 572 989 B1 décrit un procédé d'obtention et de culture de cellules de lignées de pDC à partir de cellules de ces leucémies. Ce brevet décrit tout particulièrement la lignée de pDCs humaines, dénommée GEN2.2, déposée à la CNCM (Collection Nationale de Cultures de Microorganismes, Institut Pasteur, 25 rue du Docteur Roux, F-75015 Paris) le 24 septembre 2002 sous le numéro CNCMI-2938 suivant la règle 6. 1 du Traité de Budapest, et la lignée de cellules dendritiques plasmacytoïdes humaines, dénommée GEN3, déposée à la CNCM le 16 octobre 2003 sous le numéro CNCMI-3110 suivant la règle 6. 1 du Traité de Budapest. Ces lignées peuvent être utilisées dans une mise en oeuvre tout à fait préférée de l'invention.

Le terme « pulsé » signifie que les cellules (lignée) de pDC sont incubées avec un antigène.

Dans le cadre de la présente invention le terme « antigène » définit une molécule reconnue par des cellules du système immunitaire et capable de déclencher une réaction immunitaire à médiation cellulaire.

Les antigènes selon la présente invention peuvent être des antigènes natifs ou modifiés, tumoraux ou microbiens (notamment bactériens ou viraux), tels que des peptides, protéines, glycopeptides, glycoprotéines, protéines phosphorylées.

Les antigènes peuvent être apportés dans le milieu de culture de la lignée de pDC ou être exprimés par la lignée de pDC transfectée par un vecteur permettant l'expression dudit antigène.

Dans une mise en oeuvre préférée de l'invention les antigènes sont des peptides susceptibles d'être obtenus à partir de protéines antigéniques d'origine tumorale ou virale.

Dans une mise en oeuvre particulière de l'invention les peptides susceptibles d'être obtenus à partir d'antigènes tumoraux peuvent être choisis parmi les peptides compris dans la séquence des antigènes CEA, NY-BR1 , Her-2/Neu, PSA, RAGE-1 , PRAME, TRP-2, MAGE-A1 , MAGE-A2, MAGE-A4, MAGE-A9, MAGE-A10, MAGE-C2, MUC-1 ,p53, hTERT, survivin, melan-A/MART-1 (SEQ ID No. 1 ), GP100 (SEQID No. 2), tyrosinase (SEQ ID No. 3), MAGE-A3 (SEQ ID No. 4) ou NY-ESO1 (SEQ ID No. 5), modifiés ou non.

Dans une autre mise en oeuvre de l'invention, les peptides susceptibles d'être obtenus à partir d'antigènes viraux peuvent être choisis parmi les peptides compris dans la séquence des antigènes env, nef, gp41 , gp120, gag (SEQ ID No. 6) ou pol (SEQ ID No. 7) du virus HIV, HBc ou HBs du virus HBV, core, NS3 ou NS5 du virus HCV, Flu M1 (SEQ ID No. 8) du virus influenza, CMVpp65 (SEQ ID No. 9) du virus CMV, BMLF1 (SEQ ID No. 10), LMP2 (SEQ ID No. 11), EBNA-2 (SEQ ID No. 12) ou EBNA-3a (SEQ ID No. 13) du virus EBV, modifiés ou non.

Dans le cadre de la présente invention le terme « effecteur spécifique » s'applique aux cellules de l'immunité capables de reconnaître un antigène spécifique ou un produit issu de cet antigène.

Dans une mise en oeuvre particulière de l'invention, les effecteurs spécifiques sont des effecteurs cytotoxiques et de façon tout à fait particulière ces effecteurs cytotoxiques sont des lymphocytes T spécifiques de l'antigène utilisé, et notamment CD8+.

La présente invention a également pour objet l'utilisation d'une lignée de pDC incubée avec au moins un antigène d'origine tumorale, microbienne, bactérienne ou virale et irradiée pour la fabrication d'un médicament pour la prévention et/ou le traitement de maladies infectieuses ou de cancers. Ainsi, la présente invention a également pour objet : une composition pharmaceutique ou non comprenant une lignée de pDC pulsée par incubation avec un antigène tumoral ou un antigène viral, ledit antigène viral étant choisi parmi les peptides compris dans la séquence des antigènes env, nef, gp41 , gp120, gag ou pol du virus HIV, HBc ou HBs du virus HBV, core, NS3 ou NS5 du virus HCV, flu M1 du virus influenza, pp65 du virus CMV, BMLF1 , LMP2, EBNA-2 ou EBNA-3a du virus EBV et irradiée ; une composition comprenant une culture de cellules, ladite culture comprenant une lignée de pDC incubée avec au moins un antigène et irradiée ainsi que des PBMC, lesdites pDC et PBMC partageant au moins un allèle du complexe majeur d'histocompatibilité (CMH).

Ladite lignée peut être présentée sous la forme d'un kit comprenant également des PBMC, les pDC et les PBMC partageant au moins un allèle du CMH. Le kit, dans une forme particulière peut également comprendre également au moins un antigène.

L'invention a encore pour objet une lignée de pDC incubée avec au moins un antigène et irradiée pour son utilisation vaccinale comme agent d'activation du système immunitaire. Ledit vaccin pouvant être administré dans le cadre d'une méthode de vaccination.

La demande décrit également une méthode de prévention et/ou de traitement des cancers et/ou maladies infectieuses caractérisée en ce qu'on injecte une lignée de pDC irradiée et pulsée dans un organisme, les PBMC dudit organisme et les pDC partageant au moins un allèle du complexe majeur d'histocompatibilité (CMH).

La demande décrit également une méthode de prévention et/ou de traitement des cancers et/ou maladies infectieuses caractérisée en ce qu'on injecte les effecteurs spécifiques obtenus par incubation d'une lignée de pDC avec au moins un antigène, les pDC pulsées étant ensuite irradiées et mises en contact avec des PBMC, et cultivées, les pDC et les PBMC partageant au moins un allèle du complexe majeur d'histocompatibilité (CMH).

Parmi les maladies infectieuses on peut citer les maladies dont l'agent est un microorganisme tel qu'une bactérie, un champignon, une levure ou encore un virus et plus particulièrement les infections dues aux virus influenza (ex : grippe), VIH (ex : SIDA), CMV (cytomégalovirus), EBV (Virus Epstein Barr ; ex : mononucléose), HBV et HCV(ex : hépatites), et les maladies dues aux virus émergents.

Parmi les cancers susceptibles d'être traités de manière prophylactique ou thérapeutique, on peut citer le mélanome, le cancer du sein, le cancer du poumon ou encore le cancer de la prostate, et les cancer viro-induits.

L'invention sera décrite dans certains de ces aspects de façon détaillée dans les exemples suivants.

### Description des figures

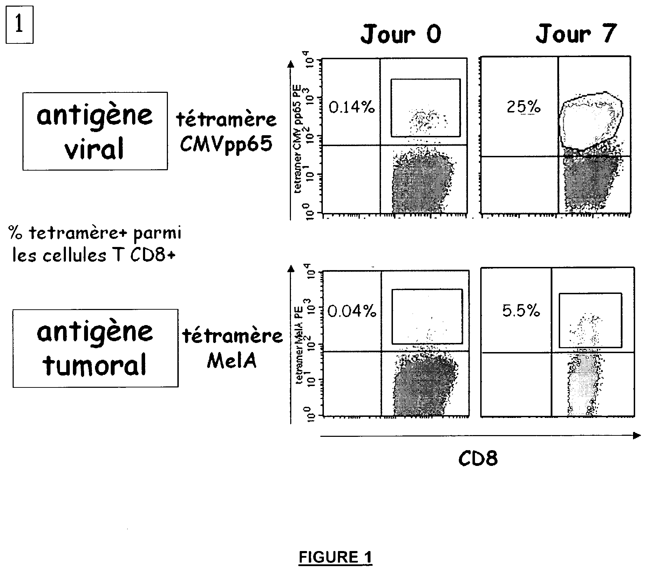

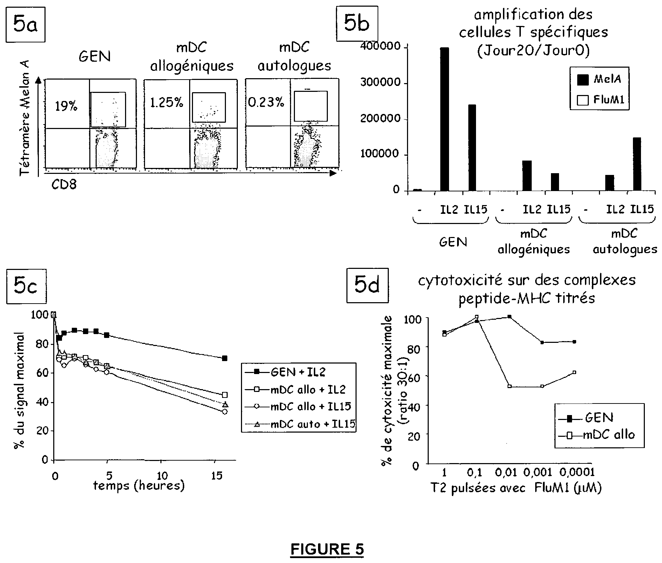

**Figure 1** **: induction de réponses T CD8 spécifiques primaires et mémoires *in vitro***
   Analyse par cytométrie de flux du pourcentage de cellules T CD8+ spécifiques à Jour (J) 0 et à J7 de la culture de PBMC issus de donneurs sains avec la lignée de pDC GEN2.2 pulsée avec un peptide (respectivement viral CMV pp65 et tumoral MelA) en utilisant des tétramères. Les pourcentages indiqués correspondent aux pourcentages de cellules tétramère+ parmi les cellules T CD8+.

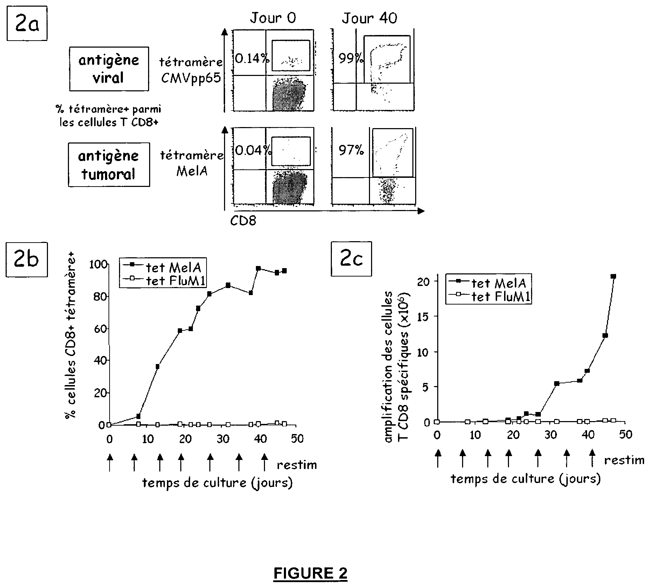

**Figure 2** **: Efficacité des stimulations répétées pour amplifier des cellules T CD8 spécifiques**
   **2a)** Analyse par cytométrie de flux des cellules T CD8+ à J0 et à J40 de la culture de PBMC issus de donneurs sains avec la lignée de pDC GEN2.2 pulsée avec les peptides CMVpp65 ou MelA après stimulations hebdomadaires en présence d'IL-2. Les pourcentages indiqués correspondent aux pourcentages de cellules tétramère+ parmi les cellules T CD8+.
   **2b)** Evolution du pourcentage de cellules T CD8+ tétramère+ et
   **2c)** Amplification (x10⁶) du nombre absolu de cellules T CD8 spécifiques de MelA après stimulation hebdomadaire de PBMC avec les pDC pulsées avec MelA en présence d'IL-2 (le tétramère FluM1 est montré en contrôle). Les flèches indiquent les re-stimulations effectuées (restim).

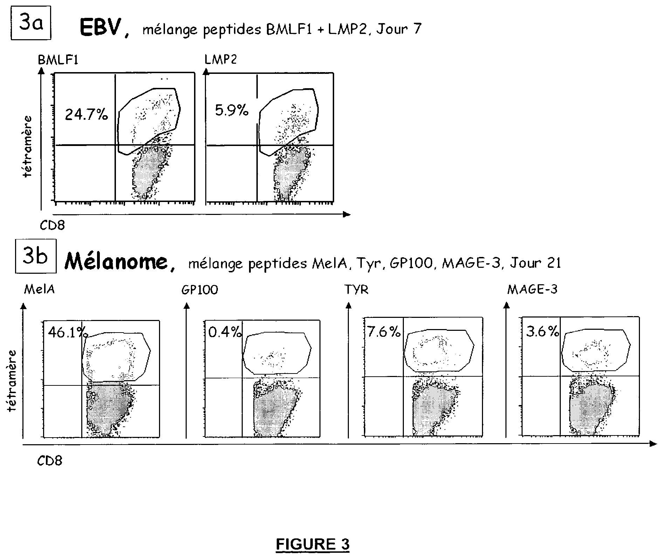

**Figure 3** **: induction simultanée des réponses T CD8 multi-spécifiques**
   Analyse par cytométrie de flux de la spécificité des cellules T CD8+ à J7 (3a) ou à J21 (3b) de la culture de PBMC issus de donneurs sains avec la lignée de pDC GEN2.2 pulsée avec un mélange de différents peptides dérivés d'antigènes viraux (issus du virus EBV : BMLF1 et LMP2, fig 3a) ou tumoraux (mélanome : MelA, GP100, TYR, MAGE-3, fig 3b). Les pourcentages indiqués correspondent aux pourcentages de cellules tétramère+ parmi les cellules T CD8+.

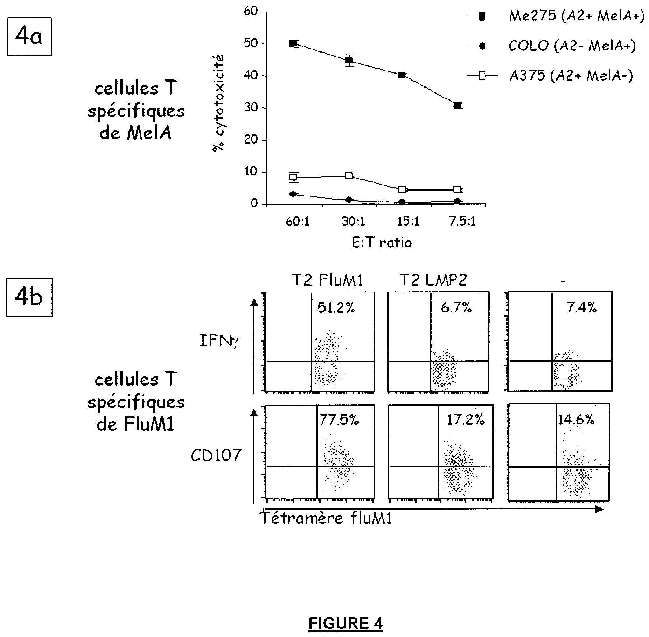

**Figure 4** **: Les cellules T CD8 spécifiques générées sont fonctionnelles *in vitro* de façon HLA- et antigène restreinte**
   4a) Restriction antigénique et par HLA-A2 de l'activité cytotoxique des cellules T CD8 spécifiques de MelA générées par culture de PBMC (donneur HLA-A2) avec la lignée de pDC GEN2.2 pulsée et irradiée vis-à-vis de lignées de mélanome: les lignées Me275 et A375 sont HLA-A2, les lignées Me275 et COLO expriment l'antigène MelA.
   **4b)** Sécrétion d'IFNγ et expression surfacique de CD107 des cellules T CD8 spécifiques de FluM1 générées par la lignée de pDC GEN2.2 pulsées et irradiées après re-stimulation (panels de gauche et au centre) ou non (panel de droite ; -) sur des cellules T2 pulsées avec le peptide FluM1 ou un peptide contrôle (LMP2) ; ratio effecteur/cible (E/T) = 10/1
**Figure 5** **: Comparaison de la lignée de pDC pulsée et irradiée selon l'invention avec des DCs myéloïdes allogéniques ou autologues pour induire des cellules T CD8 spécifiques de forte affinité et forte avidité**
   **5a)** Analyse par cytométrie de flux des cellules T CD8+ à J20 de la culture de PBMC issus de donneurs sains avec respectivement la lignée de pDC GEN2.2 (GEN), des mDC allogéniques ou autologues pulsées avec le peptide MelA après des stimulations hebdomadaires en absence de cytokines. Les pourcentages indiqués correspondent aux pourcentages de cellules tétramère+ parmi les cellules T CD8+.
   **5b)** Amplification du nombre absolu de cellules T CD8+ spécifiques de MelA à J20 de la culture de PBMC issus de donneurs sains avec respectivement la lignée de pDC GEN2.2 (GEN), des mDC allogéniques ou autologues pulsées avec le peptide MelA après des stimulations hebdomadaires en absence (-) ou en présence des cytokines IL-2 ou IL-15.
   **5c)** Comparaison de l'affinité des cellules T CD8 spécifiques générées respectivement avec la lignée de pDC (GEN2.2 ), des mDC allogéniques (mDC allo)ou autologues(mDC auto) mesurée par la dissociation du tétramère en fonction du temps après marquage.
   **5d)** Comparaison de l'avidité des cellules T CD8 spécifiques générées respectivement avec la lignée de pDC GEN2.2 (GEN) ou des mDC allogéniques (mDC allo) mesurée par leur activité cytotoxique vis-à-vis des cellules T2 pulsées avec des concentrations de peptide décroissantes. Les résultats de c et d sont exprimés en pourcentage du signal maximum obtenu (marquage maximum avec le tétramère et cytotoxicité maximale respectivement).

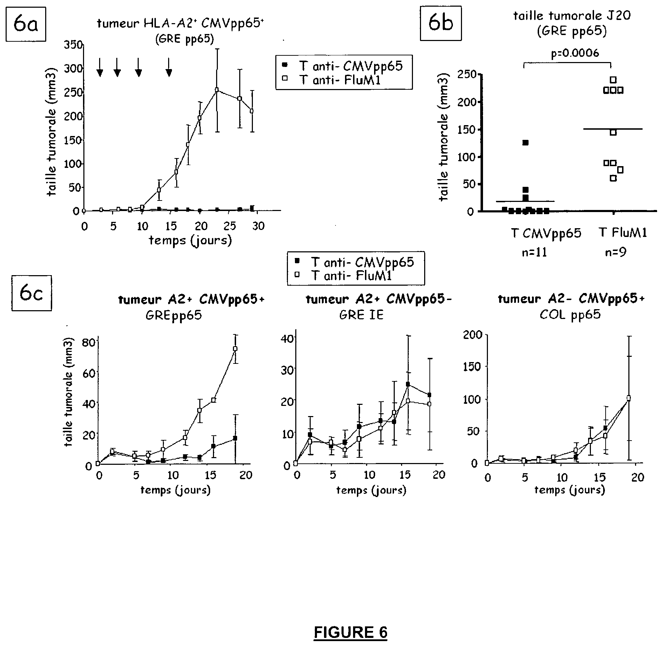

**Figure 6** **: Les cellules T CD8 spécifiques générées par la lignée de pDC pulsée selon l'invention sont fonctionnelles *in vivo***
   **6a)** Evolution de la croissance tumorale de lignées humaines HLA-A2+ et CMVpp65+ implantées dans des souris immuno-déficientes NOD-SCID b2m-^{/-} après transfert adoptif intratumoral de cellules T CD8 spécifiques de CMVpp65 ou de FluM1 amplifiées par la lignée de pDC GEN2.2 pulsée et irradiée à partir de PBMC d'un donneur HLA-A2+. Une expérience représentative est présentée. Les flèches indiquent les injections de cellules T spécifiques.
   **6b)** Comparaison de la taille des tumeurs 20 jours après leur implantation. Chaque point représente une souris humanisée.
   **6c)** Restriction antigénique et par le HLA de l'efficacité thérapeutique de cellules T CD8 spécifiques générées par la lignée de pDC pulsée irradiée selon l'invention après leur transfert adoptif. Evolution de la croissance tumorale de lignées humaines HLA-A2+ ou HLA-A2- exprimant ou non l'antigène CMVpp65+ implantées dans des souris immuno-déficientes NOD-SCID b2m^{-/-} après le transfert adoptif par voie intratumorale de cellules T CD8 spécifiques de CMVpp65 ou de FluM1 amplifiées par la lignée de pDC pulsée et irradiée selon l'invention à partir des PBMC d'un donneur HLA-A2.

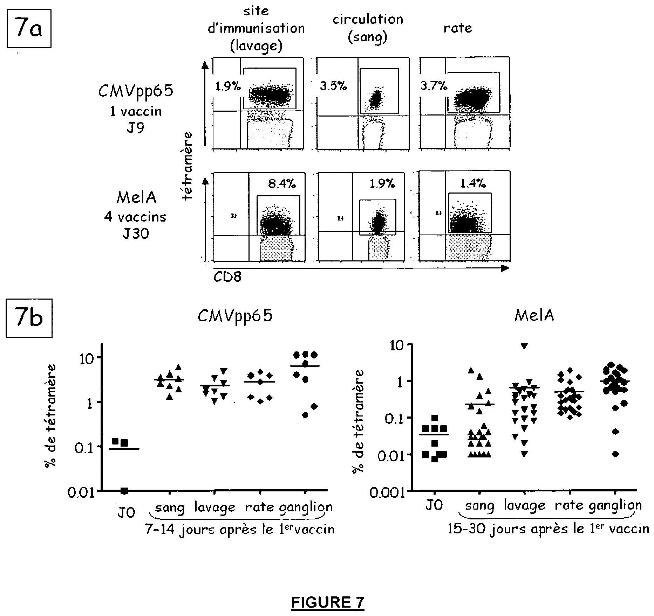

**Figure 7** **: Réponse à la vaccination par la lignée de pDC pulsée et irradiée selon l'invention dans un modèle de souris humanisées**
   **7a)** Les souris immunodéficientes NOD-SCID b2m^{-/-} ont été reconstituées par voie intrapéritonéale avec des PBMCs HLA-A2 humains et vaccinées une fois par semaine avec la lignée de pDC pulsée et irradiée selon l'invention par la même voie. Les graphes présentent les cellules T CD8+ tétramère+ au site de vaccination (site immunisation (lavage)), dans la circulation (sang) et dans les organes lymphoïdes des animaux vaccinés (rate) avec la lignée de pDC pulsées avec les peptides CMVpp65 ou MelA respectivement 9 (J9) et 30 jours (J30) après le premier vaccin. Les pourcentages indiqués correspondent aux pourcentages de cellules tétramère+ parmi les cellules T CD8+.
   **7b)** Pourcentages de cellules T CD8+ spécifiques avant (J0) et après vaccination avec la lignée de pDC pulsée avec les peptides CMVpp65 ou MelA et irradiée (après 1 vaccin et 3 rappels) dans différents organes des souris humanisées. Chaque point représente une souris humanisée.

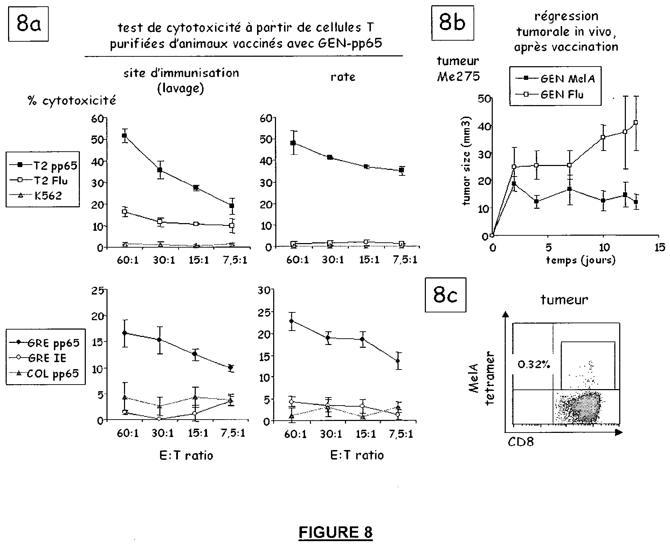

**Figure 8** **: Efficacité thérapeutique des cellules T CD8 générées après vaccination par la lignée de pDC pulsée et irradiée selon l'invention dans un modèle de souris humanisées**
   **8a)** Activité cytotoxique ex-vivo de cellules T CD8 purifiées à partir du lavage péritonéal (panels de gauche) et de la rate (panels de droite) de souris humanisées, vaccinées avec la lignée GEN2.2 pulsée avec le peptide CMVpp65 (GEN-pp65) puis irradiée, vis-à-vis de cellules T2 pulsées avec le peptide CMVpp65 ou un peptide irrelevant (panels supérieurs) et vis-à-vis de lignées HLA-A2+ ou HLA-A2- exprimant ou non l'antigèneCMVpp65 (panels inférieurs). GRE est HLA-A2+, COL est HLA-A2 négative.
   **8b)** Evolution de la croissance tumorale d'une lignée de mélanome HLA-A2+ MeIA+ implantée dans des souris immunodéficientes NOD-SCID b2m^{-/-} humanisées et vaccinées avec la lignée de pDC GEN2.2 (GEN) pulsée avec les peptides MelA ou FluM1, et irradiée.
   **8c)** Analyse par cytométrie de flux de suspensions tumorales montrant la présence de cellules T CD8 spécifiques de MelA au niveau du site tumoral d'une souris humanisée vaccinée. Le pourcentage indiqué correspond au pourcentage de cellules tétramère+ parmi les cellules T CD8+.

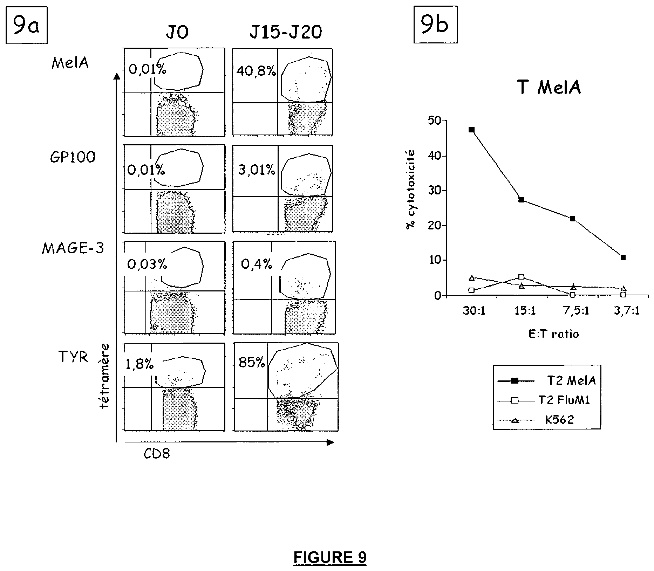

**Figure 9** **: la lignée de pDC pulsée et irradiée selon l'invention permet d'induire et d'amplifier des cellules T CD8 spécifiques et fonctionnelles chez des patients atteints de mélanome**
   **9a)** Analyse par cytométrie de flux des cellules T CD8+ à J0 et à J15-20 de la culture de PBMC, issus de patients HLA-A2+ atteints de mélanome, avec la lignée de pDC pulsée avec des peptides dérivés des antigènes MelA, GP100, MAGE3 et tyrosinase(TYR), et irradiée. Les pourcentages indiqués correspondent aux pourcentages de cellules tétramère+ parmi les cellules T CD8+.
   **9b)** Activité cytotoxique des cellules T CD8+ spécifiques de MelA générées par la lignée de pDC pulsée selon l'invention, à partir de PBMC issus de patients HLA-A2+ atteints de mélanome, vis-à-vis des cellules T2 pulsées avec le peptide MelA ou un peptide contrôle.

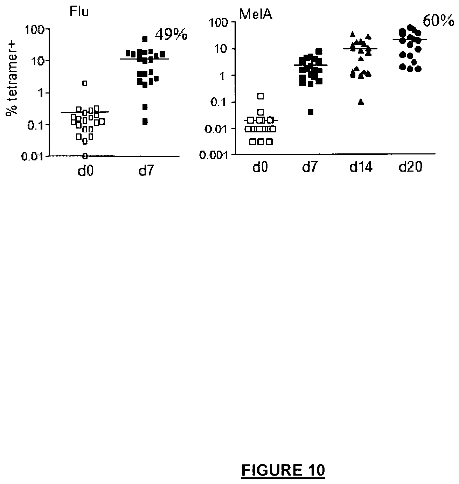

**Figure 10** **: induction de réponses T CD8 spécifiques primaires et mémoires *in vitro***
   Pourcentages de cellules T CD8+ spécifiques à Jour (J) 0 et à différents temps de la culture de PBMC issus de donneurs sains avec la lignée de pDC GEN2.2 pulsée avec un peptide (respectivement viral Flu M1 et tumoral MelA), analysés par cytométrie de flux en utilisant des tétramères. Les pourcentages indiqués correspondent aux pourcentages de cellules tétramère+ parmi les cellules T CD8+. Chaque point représente les résultats obtenus avec un donneur (n=20 pour Flu et n=18 pour MelA). La moyenne est représentée par une barre horizontale, les pourcentages indiqués sont les maxima atteints.

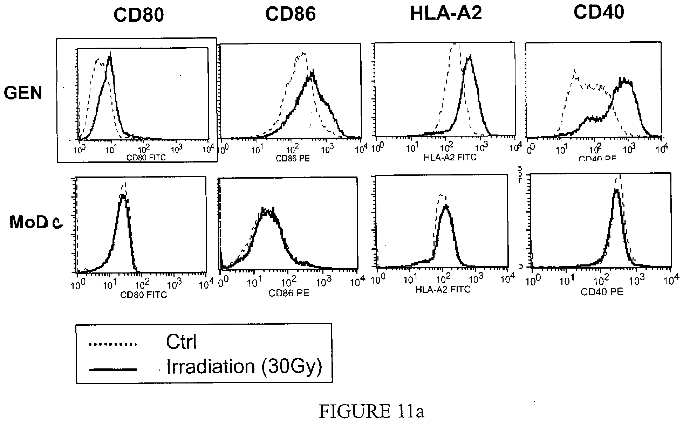

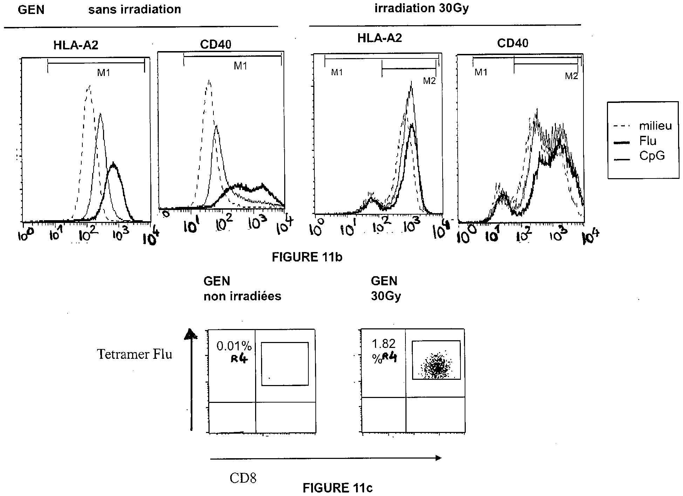

**Figure 11** **: Effet de l'irradiation sur l'activation de la lignée de pDC et sur sa capacité à induire une réponse T spécifique**
   11a) Analyse par cytométrie de flux du niveau d'expression des molécules HLA-A2 et des molécules de stimulation à la surface de la lignée de pDC irradiée ou non, puis cultivée pendant 24h, en comparaison avec des DC myéloïdes traitées dans les mêmes conditions.
   11b) Analyse par cytométrie de flux du niveau d'expression des molécules HLA-A2 et CD40 à la surface de la lignée de pDC irradiée ou non, en présence de ligands de TLR7 (virus influenza inactivé : Flu) ou de TLR9 (CpGA), puis cultivées pendant 24h.
   11c) Analyse comparative par cytométrie de flux du pourcentage de cellules T CD8+ spécifiques après 7 jours de culture de PBMC issus de donneurs sains avec la lignée de pDC GEN2.2 irradiée ou non et pulsée avec le peptide Flu. Les pourcentages indiqués correspondent aux pourcentages de cellules tétramère+ parmi les cellules T CD8+.

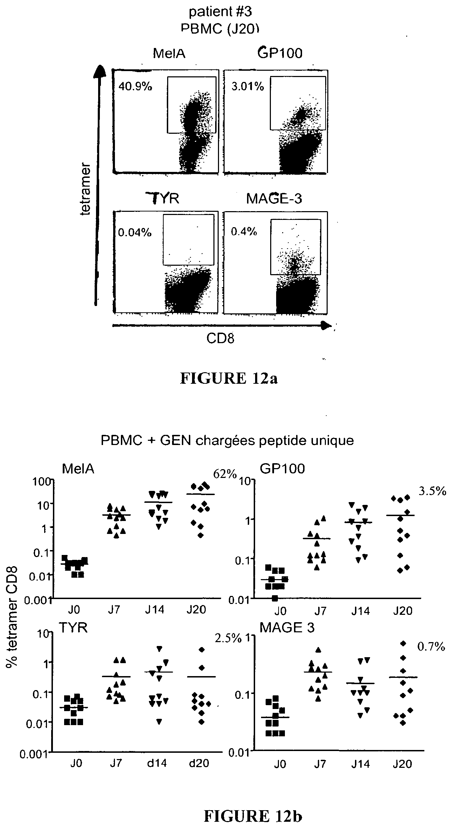

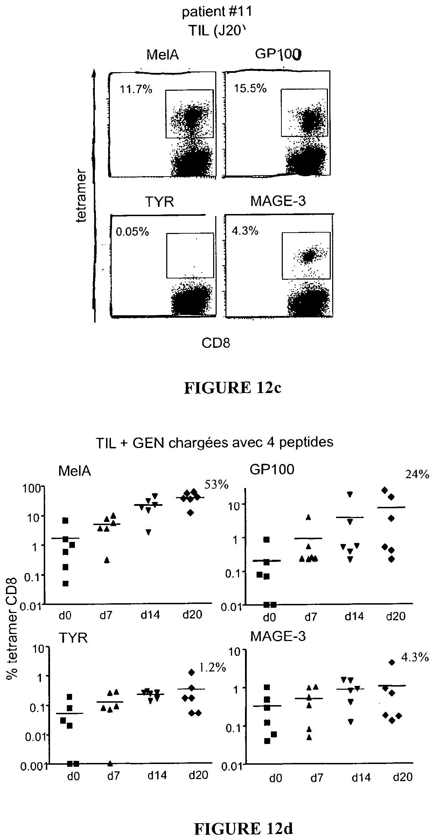

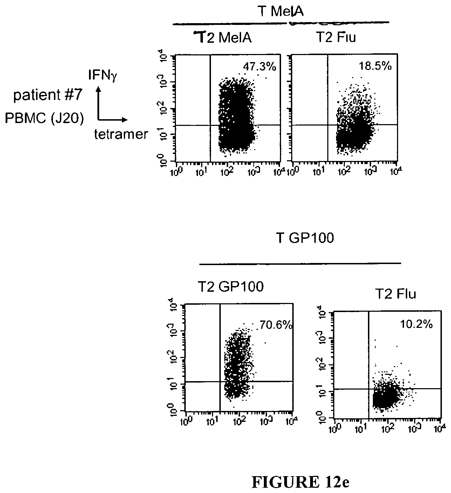

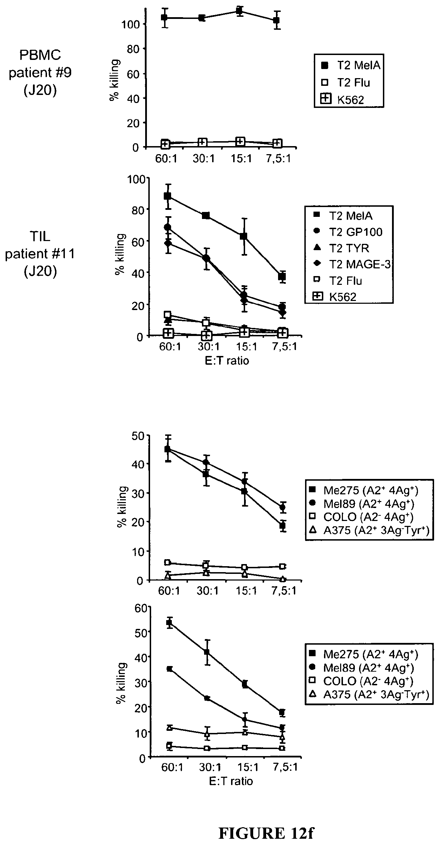

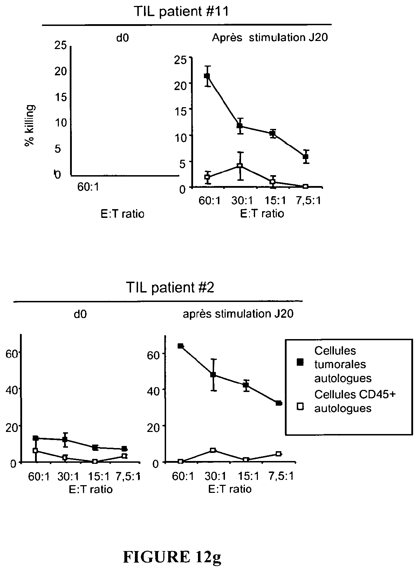

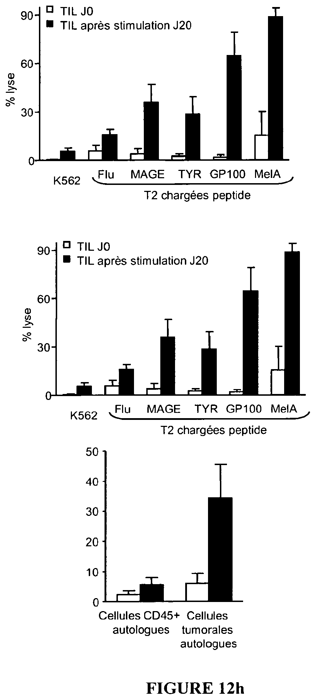

**Figure 12** **: Efficacité de la lignée de pDC pour amplifier ex-vivo des cellules T CD8+ antitumorales spécifiques à partir de PBMC et TIL de patients porteurs d'un mélanome stade I-IV.**
   Les PBMC (n=12) et TIL (n=6) provenant de patients porteurs de mélanome (stades I à IV) ont été cultivés avec la lignée de pDC pulsée avec un peptide (a-b) ou un mix de quatre peptides tumoraux (MelA, GP100, TYR ou/et MAGE-3) (c-d), et irradiée. Les cultures ont été restimulées chaque semaine, en présence d'IL2.
   a-d) Analyse par cytométrie de flux du pourcentage de cellules T CD8+ tétramère+ au cours de cultures (20 jours) des PBMC (a, b) ou des TIL (c, d) avec la lignée de pDC pulsée et irradiée. Une expérience représentative réalisée avec des PBMC (a) ou des TIL (c) est montrée (analyse à J20). Les résultats de l'ensemble des expériences réalisées sont montrés pour les PBMC (b) et pour les TIL (d), avec une mesure des pourcentages de CD8+ tétramères+ initiale (J0) et après 7, 14 et 20 jours de culture. Chaque point représente un patient, les barres horizontales représentent la moyenne, les pourcentages indiqués sont les maxima atteints.
   e-h) Analyse de la fonctionnalité et de la spécificité des lymphocytes T antitumoraux induits par la lignée de pDC pulsée et irradiée, à partir des PBMC et TIL. e) Mesure de la sécrétion d'IFNγ par les lymphocytes T générés à partir de PBMC activés par la lignée de pDC pulsée par les peptides MelA ou GP100 par cytométrie de flux, après restimulation par des cellules T2 pulsées avec le peptide relevant ou un peptide contrôle (expérience représentative de 8 réalisées avec PBMC (n=6) et TIL (n=2). Les pourcentages indiqués correspondent aux pourcentages de cellules T CD8+ tétramères+ produisant de l'IFNγ.
   f-h) Mesure de la cytotoxicité (relargage de ⁵¹Cr) des lymphocytes T générés. Les lymphocytes T purifiés après 15 à 20 jours de culture à partir de PBMC ou de TIL ont été utilisés comme effecteurs dans un test de cytotoxicité contre la lignée T2 pulsée, des cellules de mélanome allogéniques et autologues, ainsi que des cellules non tumorales CD45+ autologues. f) les PBMC (patient #9) ont été activés par la lignée de pDC pulsée avec le peptide MelA, et les TIL (patient #11) ont été activés par la lignée de pDC pulsée avec un mélange des quatre peptides MelA, GP100, TYR et MAGE-3. Ces expériences sont représentatives de 12 réalisées avec des PBMC et 6 réalisées avec des TIL. g) Pourcentage de lyse des cellules tumorales autologues en comparaison avec les cellules non tumorales CD45+ autologues initialement et après stimulation de TIL. Ces deux expériences sont représentatives de six échantillons de TIL analysés. h) Comparaison de l'efficacité de lyse des cibles indiquées par les TIL avant et après stimulation par la lignée de pDC pulsée avec un mélange de quatre peptides tumoraux. La moyenne +/- EC des pourcentages de lyse mesurés au ratio 60 :1 est montrée (n=6).

### EXEMPLES

### Exemple 1 - la lignée de pDC GEN 2.2 permet l'induction de réponses T CD8 primaires et mémoires spécifiques d'un antigène d'intérêt in vitro à partir de cellules de sujets sains

La capacité des cellules GEN2.2 à induire une réponse T CD8 spécifique d'antigène en situation semi-allogénique a été testée en cultivant des cellules de la lignée GEN2.2 (ci-après : cellules GEN2.2), pulsées avec un peptide d'intérêt et irradiées, avec des cellules mononuclées du sang périphériques (PBMC) de donneurs volontaires sains HLA-A2+.

Pour pulser les cellules GEN2.2 avec un peptide d'intérêt, celles-ci sont lavées 3 fois en milieu RPMI complet (RPMI 1640 Glutamax supplémenté par 1 mM pyruvate de sodium, 20 µg/ml gentamicine, 100 µM acides aminés non essentiels) sans SVF et resuspendues à 1.10⁶ /ml. Les cellules sont additionnées de 100 ng/ml de ß2-microglobuline et incubées 10min à 37°C (étuve). Le peptide d'intérêt est alors ajouté à raison de 10µM. La suspension cellulaire est ensuite incubée 3h à 37°C (étuve) avec agitation régulière. Les cellules sont ensuite lavées, irradiées à 30 Gy et resuspendues à 2.10⁵ / ml en milieu RPMI complet additionné de 10% de sérum de veau foetal. Les PBMC sont purifiées par Ficoll à partir d'une poche de sang d'un donneur sain. Elles sont resuspendues à 2.10⁶ / ml en milieu RPMI complet additionné de 10% de sérum de veau foetal. Les cellules GEN2.2 pulsées et irradiées sont cocultivées avec les PBMC semi-allogéniques HLA-A2+ en plaque 24 puits (2.10⁵ GEN2.2 + 2.10⁶ PBMC / 2ml) pendant 7 jours à 37°C. Le phénotype des cellules T CD8 est réalisé au début de l'expérimentation (J0) et après 7 jours de culture (J7). La spécificité des cellules T CD8 est analysée grâce à un marquage par des tétramères (Figure 1).

La coculture de PBMC avec la lignée de pDC GEN2.2 pulsée avec un peptide dérivé d'antigènes viraux ou tumoraux et irradiée permet d'induire et d'amplifier des cellules T CD8 spécifiques du peptide d'intérêt avec une grande efficacité en seulement 7 jours de culture. Nous avons démontré l'induction de réponses primaires dirigées contre les antigènes tumoraux MelA, GP100, MAGE-3, tyrosinase et NY-ESO1, et de réponses mémoires dirigées contre les antigènes viraux FluM1, CMVpp65, EBV BMLF1, EBV LMP2 en utilisant cette stratégie.

### Exemple 2 - la lignée de pDC pulsée et irradiée permet l'amplification massive de cellules T CD8 spécifiques d'un antigène d'intérêt

Pour optimiser l'induction de cellules T spécifiques, les cellules ont été restimulées régulièrement avec les cellules GEN2.2 pulsées et irradiées, en présence d'IL2. Pour cela, les cellules sont mises en culture comme décrit précédemment. Après 7 jours de culture, les cellules sont récupérées, lavées, comptées et suspendues à 2.10⁶ / ml en milieu RPMI complet additionné de 10% de sérum de veau foetal. Elles sont remises en culture en plaque 24 puits avec des cellules GEN2.2 pulsées et irradiées à raison de 2.10⁵ / ml en présence d'IL2 à 200 U/ml. Ce processus est renouvelé tous les 7 jours. Le pourcentage et le nombre absolu de cellules T CD8 spécifiques du peptide utilisé sont évalués avant chaque restimulation.

Comme le montre la Figure 2, des stimulations répétées par la lignée GEN2.2 pulsée irradiée en présence de cytokines (IL-2) permettent l'amplification massive de cellules T CD8 spécifiques du peptide utilisé avec une très grande efficacité. Ceci peut conduire à l'amplification exclusive des cellules T CD8 spécifiques du peptide d'intérêt (obtention de plus de 97% de cellules T CD8 tétramère+) et à leur amplification concomitante (multiplication par un facteur 20.000.000 du nombre absolu de cellules T CD8 spécifiques) à la fois pour des antigènes viraux et tumoraux.

### Exemple 3 - la lignée de pDC GEN2.2 permet l'induction simultanée de réponses T CD8 primaires ou mémoires multi-spécifiques

Pour évaluer la possibilité d'induire des réponses T multi-spécifiques, les cellules de la lignée GEN2.2 ont été pulsées simultanément avec plusieurs peptides dérivés d'antigènes viraux (BMLF1, LMP2) ou d'antigènes tumoraux (MelanA, GP100, tyrosinase, MAGE-3), puis irradiées. Elles ont ensuite été cultivées avec des PBMC semi-allogéniques HLA-A2+, selon les protocoles décrits précédemment. Au sein de ces PBMC, les pourcentages initiaux de cellules tétramère+ (BMLF1, LMP2, MelA, GP100, TYR, ou MAGE-3) parmi les lymphocytes T CD8+ étaient strictement inférieurs à 0,05%. L'amplification de cellules T CD8 spécifiques de chacun des peptides utilisés est analysée grâce à un marquage tétramère à 7 jours (pour EBV, les peptides BMLF et LMP2, Figure 3a) ou à 21 jours de culture avec deux restimulations (pour MelA, GP100, TYR et MAGE-3, Figure 3b).

L'utilisation de la lignée de pDC pulsée avec un mélange de différents peptides permet donc d'induire simultanément des cellules T CD8 spécifiques de chacun des peptides utilisés, à la fois dans des modèles viraux et tumoraux.

### Exemple 4 - les cellules T spécifiques générées par la lignée de pDC GEN2.2 présentent une très bonne fonctionnalité in vitro

Trois méthodes ont été utilisées pour évaluer la fonctionnalité des cellules T CD8 générées par la lignée de pDC pulsée GEN2.2 : test de cytotoxicité, sécrétion d'IFNγ et expression membranaire de CD107 après re-stimulation spécifique.

L'activité cytotoxique des cellules T spécifiques générées par la lignée GEN2.2 est évaluée par un test de relarguage de ⁵¹Cr. Pour cela, après stimulation de PBMC semi-allogéniques avec des cellules GEN2.2 pulsées avec le peptide MelA et irradiées, les cellules sont récupérées et les lymphocytes T CD8+ sont purifiés (tri T CD8 EasySep par sélection négative) et mis en présence de cellules cibles marquées au ⁵¹Cr à différents ratios (E :T ratio allant de 60:1 à 7,5:1) pendant 4h. Un dosage du ⁵¹Cr dans le surnageant est ensuite effectué. Les cellules T anti-MelA sont par exemple capables de lyser efficacement des cellules tumorales HLA-A2+ qui expriment Melan-A (Me275) mais n'ont pas d'activité cytotoxique envers des cellules tumorales qui n'expriment pas MelA (A375) ou qui ne sont pas HLA-A2 (COLO829) (Figure 4a).

La capacité effectrice des cellules T spécifiques a été aussi évaluée par leur capacité à secréter de l'IFNγ et à exprimer CD107 en surface après re-stimulation spécifique. Pour cela après stimulation de PBMC semi-allogéniques avec des cellules GEN2.2 pulsées avec le peptide fluM1 et irradiées, les cellules sont récupérées, comptées et un marquage tétramère est réalisé (30 min à température ambiante). Après lavage, les cellules sont suspendues à 1.10⁶/200µl dans du milieu RPMI 10% SVF. Les cellules T2 pulsées avec le peptide d'intérêt (flux1) ou un peptide contrôle (LMP2) sont ajoutées ou non (ratio cellules effectrices : cellules restimulantes = 10:1). Pour la sécrétion d'IFNγ, les cellules sont restimulées pendant 5h30 à 37°C en présence de brefeldine A (GolgiPlug à 1µl/ml) pendant les 3 dernières heures. Après marquage des antigènes de surface (CD3, CD8) un marquage intracellulaire d'IFNγ est ensuite réalisé (Figure 4b). Pour mesurer l'expression de CD107, les anticorps anti-CD107a et anti-CD107b (20ml / 10⁶ cellules) sont ajoutés pendant toute la durée de la re-stimulation et les cellules sont incubées pendant 5h à 37°C en présence de monensine (GolgiSTOP à 0,67µl/ml) pendant les 4 dernières heures. Un marquage des antigènes de surface (CD3, CD8) est ensuite réalisé (Figure 4b). L'analyse du marquage d'IFNγ et de CD107 est effectuée sur les cellules T CD8+ tétramère+. Ainsi des cellules T CD8+ anti-FluM1 générées grâce à la lignée GEN sont capables de secréter de l'IFNγ et d'exprimer CD107 spécifiquement après re-stimulation par des cellules T2 pulsées avec FluM1 et pas après re-stimulation par des cellules T2 pulsées avec un autre peptide (T2 LMP2) ou en absence de re-stimulation (Figure 4b).

Les cellules T CD8 spécifiques d'un antigène générées par la lignée de pDC GEN2.2 sont donc capables de tuer des cellules cibles qui expriment cet antigène de façon restreinte par le HLA. Elles secrètent spécifiquement de l'IFNγ et expriment CD107 après re-stimulation par des cellules présentant le peptide dont elles sont spécifiques dans le bon contexte HLA.

### Exemple 5 - la lignée de pDC GEN2.2 permet d'induire et d'amplifier des cellules T CD8 spécifiques d'un antigène d'intérêt avec une plus grande efficacité que les cellules dendritiques myéloïdes (mDC) et leur confère un plus grand pouvoir fonctionnel

Nous avons comparé les capacités de la lignée de pDC GEN2.2 avec celles de cellules dendritiques myéloïdes (mDC) allogéniques ou autologues pour l'induction de cellules T CD8 spécifiques et évalué leurs propriétés fonctionnelles.

Pour générer les mDCs, des monocytes ont été purifiés par tri négatif (utilisation du kit EasySep®, suivant les recommandations du fabricant) à partir de PBMC et cultivés à 0,5.10⁶ cellules/ml en milieu RPMI 10% SVF en présence de GM-CSF (500 U/ml) et d'IL4 (10 ng/ml) pendant 6 jours. Les mDCs et les cellules GEN2.2 sont ensuite pulsées avec un peptide d'intérêt comme décrit dans l'exemple 1, irradiées et cultivées avec des PBMC HLA-A2+ semi-allogéniques ou autologues (pour les mDCs). Des re-stimulations sont effectuées tous les 7 jours en présence d'IL2 200 U/ml ou d'IL 15 (5 ng/ml). Le pourcentage (Figure 5a) et le nombre absolu (Figure 5b) de cellules T CD8 tétramère+ est évalué respectivement à 7 et 20 jours de culture après stimulation des PBMC avec des GEN2.2, des mDC allogéniques ou des mDC autologues pulsées avec MelA. La lignée GEN2.2 permet une induction beaucoup plus efficace de cellules T anti-MelA en comparaison avec les mDC allogéniques ou autologues en conditions basales ou en présence de cytokines. L'affinité des cellules T CD8 générées dans les différentes conditions est mesurée par la dissociation du tétramère à 37°C. Pour cela un marquage tétramère des cellules T CD8 spécifiques est réalisé (30 min à 4°C). Après lavage, les cellules sont incubées à 37°C. Après différents temps d'incubation (de 0 à 16h), les cellules sont récupérées et fixées pour analyse par cytométrie en flux. La dissociation du tétramère est évaluée en pourcentage du marquage tétramère initial (Figure 5c). L'avidité des cellules T CD8 générées dans les différentes conditions est mesurée par leur activité cytotoxique sur des cellules T2 pulsées avec des concentrations peptidiques décroissantes (de 1 à 0,0001 µM). La cytotoxicité est exprimée en pourcentage de la cytotoxicité maximale observée (Figure 5d). Les cellules T anti-MelA générées par la lignée GEN2.2 présentent une plus forte affinité et une plus grande avidité par rapport aux cellules T anti-MelA générées par les mDCs allogéniques ou autologues.

La lignée de pDC GEN2.2 est beaucoup plus efficace que les mDCs pour induire des cellules T spécifiques (obtention d'un plus grand pourcentage de cellules tétramère+ dans les même conditions) et surtout pour amplifier les cellules T spécifiques (amplification dix fois plus intense avec les pDC par rapport aux mDC). De plus, la lignée GEN2.2 confère aux cellules T spécifiques un plus grand pouvoir fonctionnel car elles acquièrent une plus grande affinité et une plus forte avidité que les cellules générées avec les mDC.

### Exemple 6 - les cellules T spécifiques générées par la lignée de pDC présentent une très bonne activité antitumorale in vivo par transfert adoptif

L'efficacité fonctionnelle des cellules T spécifiques générées *in vitro* par la lignée de pDC GNE2.2 a été évaluée *in vivo* par leur capacité à inhiber le développement d'une tumeur humaine implantée chez une souris immuno-déficiente.

Une tumeur est établie chez des souris NOD-SCID b2m-/- par injection de 1 à 2,5.10⁶ cellules tumorales par voie sous-cutanée dans le flanc. Parallèlement, des cellules T CD8 spécifiques d'un antigène d'intérêt sont générées in vitro par la lignée GEN2.2 comme décrit précédemment. Après sélection des cellules T CD8+ par tri négatif (utilisation du kit EasySep® selon les recommandations du fabricant), 1 à 5.10⁶ cellules T CD8+ sont ensuite transférées par voie intra-tumorale à raison de 4 injections espacées de 4-5 jours. L'évolution de la croissance tumorale est ensuite mesurée. La Figure 6a montre l'évolution de la croissance de la tumeur GRE pp65 (HLA-A2+CMVpp65+) après transfert adoptif de cellules T CD8+ anti-CMVpp65 ou anti-FluM1. La Figure 6b montre la comparaison des tailles tumorales 20 jours après leur implantation après traitement par des cellules T CD8+ anti-CMVpp65 ou anti-FluM1 (chaque point représente une souris).

Les cellules T anti-CMVpp65 générées in vitro par la lignée GEN permettent donc d'inhiber le développement d'une tumeur HLA-A2+CMVpp65+. L'efficacité thérapeutique des cellules injectées observée est restreinte par le HLA et l'antigène car ce traitement ne permet pas l'inhibition du développement de tumeurs qui ne sont pas HLA-A2 (COL pp65) ou qui n'expriment pas l'antigène contre lequel elles sont dirigées (GRE IE) (Figure 6c).

### Exemple 7 - la lignée de pDC permet d'induire une réponse T spécifique primaire ou mémoire in vivo par vaccination

Pour évaluer la capacité de la lignée de pDC GEN2.2 à induire une réponse T spécifique *in vivo,* un modèle de souris humanisées a été développé (souris immuno-déficientes reconstituées avec un système immunitaire humain).

Pour cela, 50.10⁶ de PBMC sont injectés par voie intra-péritonéale à des souris NOD-SCID b2m-/-. Le lendemain, 5.10⁶ cellules GEN2.2 pulsées avec un peptide d'intérêt et irradiées sont injectées par voie intra-péritonéale. La vaccination est renouvelée ou non 1 fois/semaine. A différents temps après vaccination (9 jours pour vaccin CMV, 30 jours pour vaccin MelA), l'induction de cellules T CD8+ spécifiques est évaluée au niveau du site d'immunisation (lavage), de la circulation et des organes lymphoïdes secondaires (rate, ganglions). Pour cela les souris sont soumises à un prélèvement sanguin puis sacrifiées. Un lavage du péritoine avec 15 ml de milieu RPMI permet de récupérer les cellules du site d'immunisation. Les organes lymphoïdes sont ensuite prélevés et une suspension cellulaire est réalisée après digestion à l'aide de collagénase 2,5 mg/ml. Un marquage des cellules T CD8 spécifiques par tétramère est réalisé. La Figure 7a présente un exemple obtenu avec une souris dans chaque cas, la Figure 7b présente le niveau initial de cellules T CD8 tétramère+ parmi les PBMC et l'ensemble des réponses à la vaccination CMV ou MelA obtenues. Ainsi une vaccination par lignée de pDC pulsée selon l'invention permet d'induire très efficacement des réponses T CD8 spécifiques *in vivo,* à la fois au niveau du site d'immunisation mais aussi en circulation et dans les organes lymphoïdes secondaires. Nous avons démontré une réponse à la vaccination in vivo avec les antigènes viraux FluM1, EBV BMLF1, EBV LMP2, CMVpp65 et avec les antigènes tumoraux MelA, GP100, MAGE-3, tyrosinase.

L'injection du produit (lignée de pDC GEN2.2 pulsée irradiée) *in vivo* permet d'induire des réponses T spécifiques directement chez le receveur, à la fois pour des antigènes viraux et tumoraux. Les niveaux de réponse obtenus sont élevés par rapport aux niveaux de référence connus. Ceci démontre la faisabilité d'utilisation du produit comme vaccin cellulaire. Cette efficacité apporte la preuve de concept préclinique de l'efficacité thérapeutique de ce produit comme vaccin cellulaire.

### Exemple 8 - les cellules T spécifiques générées in vivo après vaccination par la lignée de pDC présentent une très bonne fonctionnalité

Nous avons évalué l'efficacité thérapeutique de la réponse induite *in vivo* après vaccination d'une part par l'activité cytotoxique *ex-vivo* des cellules T CD8 spécifiques, et d'autre part par l'effet sur le développement d'une tumeur *in vivo.*

Les souris NOD-SCID b2m^{-/-} ont été reconstituées avec 50.10⁶ de PBMC par voie intra-péritonéale puis vaccinées avec 5.10⁶ cellules GEN2.2 pulsées avec un peptide dérivé de CMV (CMVpp65) et irradiées. Neuf jours après vaccination, les cellules T CD8+ sont purifiées à partir de prélèvements du site d'immunisation et des organes lymphoïdes (tri négatif avec le kit EasySep, utilisé selon les consignes du fabricant) et leur activité cytotoxique est évaluée contre des cellules T2 pulsées avec le peptide CMVpp65 ou un peptide contrôle (fluM1) (Figure 8a), et sur des cellules tumorales HLA-A2+ (GRE pp65) ou HLA-A2- (COL pp65) exprimant l'antigène correspondant ou non (GRE IE) (Figure 8a). Comme le montre la Figure 8a, les cellules T CD8 spécifiques générées *in vivo* après vaccination présentent une activité cytotoxique sur des cellules T2 et sur des cellules tumorales HLA-A2+ exprimant l'antigène dont elles sont spécifiques.

Pour évaluer l'efficacité thérapeutique de la vaccination par lignée de pDC pulsée selon l'invention sur le développement d'une tumeur, les souris NOD-SCID b2m^{-/-} ont été reconstituées avec 50.10⁶ cellules de PBMC par voie intra-péritonéale puis vaccinées avec 5.10⁶ cellules de la lignée GEN2.2 pulsées avec un peptide dérivé de MelA ou de Flu (FluM1) 1 fois par semaine. Cinq à dix jours après la première vaccination, 10.10⁶ cellules tumorales HLA-A2+ MelA+ (Me275) ont été implantées dans le flanc par voie sous-cutanée. L'évolution de la croissance tumorale est ensuite observée (Figure 8b). La présence de cellules T CD8 spécifiques de MelA est ensuite recherchée au niveau du site tumoral par l'analyse par cytométrie de flux d'une suspension de la tumeur 1 mois après son implantation (Figure 8c). Ainsi, la vaccination par lignée de pDC pulsée selon l'invention permet d'inhiber le développement d'une tumeur HLA-A2+ qui exprime l'antigène d'intérêt utilisé pour pulser les cellules GEN. Les cellules T CD8 spécifiques du peptide d'intérêt générées par la vaccination sont capables de migrer au niveau du site d'expression de l'antigène et de lyser les cellules tumorales.

Les exemples démontrent que les cellules T CD8 spécifiques induites *in vivo* par vaccination présentent une efficacité cytotoxique *ex-vivo* restreinte par le HLA et l'antigène, et sont capables de migrer sur le site d'expression de l'antigène dont elles sont spécifiques et d'inhiber le développement d'une tumeur.

### Exemple 9 - la lignée de pDC GEN2.2 permet l'induction et amplification de cellules T CD8 spécifiques d'antigènes d'intérêt et fonctionnelles à partir de cellules de patients atteints de cancer

Pour analyser si la lignée de pDC pulsée selon l'invention permet d'amplifier une réponse T spécifique chez des patients atteints de cancer, nous avons cultivé des PBMC issus de patients HLA-A2 atteints de mélanome stade IV avec les cellules GEN2.2 pulsées avec les peptides dérivés de MelA, GP100, MAGE-3 ou tyrosinase selon le même protocole que dans l'exemple 2. Les cellules T CD8+ spécifiques sont analysées par cytométrie de flux avant stimulation et après 3 stimulations par les cellules GEN pulsées (Figure 9a). La fonctionnalité des cellules T CD8 générées a ensuite été évaluée en mesurant leur activité cytotoxique sur des cellules T2 pulsées avec le peptide d'intérêt ou un peptide contrôle (FluM1) (Figure 9b) selon le même protocole que dans l'exemple 4.

La lignée de pDC GEN2.2 permet donc d'induire une amplification massive de cellules T CD8 spécifiques de différents antigènes tumoraux à partir des cellules de patients atteints de cancer. L'activité cytotoxique des cellules T générées confirme leur fonctionnalité antitumorale.

### Exemple 10: La lignée de pDC GEN2.2 permet l'induction de réponses T CD8 primaires et mémoires spécifiques d'un antigène d'intérêt chez tous les donneurs testés.

La capacité des cellules GEN2.2 à induire une réponse T CD8+ spécifique d'antigène en situation semi-allogénique a été testée en cultivant les cellules de la lignée de pDC pulsées et irradiées en présence de PBMC de donneurs volontaires sains HLA-A*0201+. L'induction d'une réponse mémoire est évaluée en utilisant le peptide MelanA, l'induction d'une réponse mémoire est évaluée à l'aide du peptide flu M1 de la matrice d'influenza.

Pour pulser les cellules GEN2.2 avec un peptide d'intérêt, celles-ci sont lavées 3 fois en milieu RPMI complet (RPMI 1640 Glutamax supplémenté par 1 mM pyruvate de sodium, 20 µg/ml gentamicine, 100 µM acides aminés non essentiels) sans SVF et resuspendues à 1.10⁶ /ml. Les cellules sont additionnées de 100 ng/ml de ß2-microglobuline et incubées 10min à 37°C (étuve). Le peptide d'intérêt est alors ajouté à raison de 10µM (Flu M1) ou 1µM (MelA). La suspension cellulaire est ensuite incubée 3h à 37°C (étuve) avec agitation régulière. Les cellules sont ensuite lavées, irradiées à 30 Gy et resuspendues à 2.10⁵ / ml en milieu RPMI complet additionné de 10% de sérum de veau foetal. Les PBMC sont purifiées par Ficoll à partir de poches de sang de donneurs sains. Elles sont resuspendues à 2.10⁶/ml en milieu RPMI complet additionné de 10% de sérum de veau foetal. Les cellules GEN2.2 pulsées et irradiées sont cocultivées avec les PBMC semi-allogéniques HLA-A2+ en plaque 24 puits (2.10⁵ GEN2.2 + 2.10⁶ PBMC/2ml) pendant 7 à 20 jours à 37°C. Des restimulations hebdomadaires des cultures dans le modèle Mel A sont réalisées, dans les mêmes conditions qu'initialement, avec addition d'IL-2 (200U/ml). Le phénotype des cellules T CD8 est évalué au début de l'expérimentation (J0) et après 7 jours de culture (J7) pour la mesure de la réponse anti-FluM1 et après 7, 14 et 20 jours pour la mesure de la réponse anti-MelA. La spécificité des cellules T CD8 est analysée grâce à un marquage par des tétramères (Figure 10).

Après 7 jours de culture, les pourcentages de lymphocytes T tétramères+ spécifiques de l'antigène viral Flu sont en moyenne de 11% (0.1 à 49%) (20 cultures réalisées à partir des PBMC de donneurs différents). Les pourcentages de lymphocytes T antitumoraux dirigés contre MelA atteignent en moyenne 22% (2 à 60%) après 20 jours de culture (18 cultures réalisées à partir des PBMC de donneurs différents). Les pourcentages respectifs initiaux de lymphocytes T tétramères+ anti Flu et anti MelA étaient en moyenne de 0.23 et 0.02 %.

Nous démontrons dans cet exemple qu'avec cette stratégie il est possible avec 100% des donneurs (HLA-A*0201) d'amplifier des lymphocytes T spécifiques d'antigènes, dans le contexte d'une réponse primaire ou mémoire.

### Exemple 11: L'irradiation de la lignée de pDC induit leur maturation.

Les cellules de la lignée de pDC pulsées et irradiées induisent l'amplification de Lymphocytes T spécifiques HLA-A*0201. Nous avons évalué l'effet de l'irradiation sur la maturation des pDC, en comparaison avec des cellules dendritiques myéloïdes.

Des cellules dendritiques myéloïdes (MoDC) ont été générées à partir de monocytes purifiés par tri négatif (utilisation du kit EasySep®, suivant les recommandations du fabricant) à partir de PBMC et cultivés à 0,5.10⁶ cellules/ml en milieu RPMI 10% SVF en présence de GM-CSF (500 U/ml) et d'IL4 (10 ng/ml) pendant 6 jours.

Les cellules de la lignée GEN2.2 et les cellules dendritiques myéloïdes (deux donneurs différents) ont été irradiées (30Gray) et mises en culture en milieu RPM110% SVF pendant 24h, puis phénotypées. Les niveaux d'expression des molécules CD40, CD80, CD86 et HLA-A2 ont été suivis par cytométrie de flux (Fig 11a). L'irradiation induit l'augmentation de l'expression de ces quatre molécules spécifiquement dans les pDC et pas dans les MoDC.

L'effet de l'irradiation des pDC a été comparé avec l'effet d'inducteurs forts de maturation des pDC que sont le virus de l'influenza (Flu, Ligand de TLR7) et le CpG 2336 (ligand de TLR9). Les cellules de la lignée GEN2.2 ont été irradiées (30 Gray) ou non et mises en culture en présence ou non de virus de l'influenza inactivé ou de CpG 2336, pendant 24h. Les cellules ont ensuite été phénotypées, et l'expression de HLA-A2 et CD40 ont été mesurées par cytométrie de flux. L'irradiation induit une maturation des cellules de la lignée GEN2.2 aussi importante que celle observée en présence des ligands de TLR7 ou 9 (Fig 11 b).

Pour évaluer l'influence de la maturation induite par l'irradiation des cellules GEN2.2 sur l'induction des réponses T, les cellules de la lignée GEN2.2 ont été pulsées avec le peptide Flu, puis irradiées ou non. Elles ont ensuite été cultivées avec des PBMC semi-allogéniques HLA-A2+, selon les protocoles décrits précédemment. L'amplification de cellules T CD8 spécifiques du peptide Flu est analysée grâce à un marquage tétramère à 7 jours (Figure 11c). Les pDC non-irradiées sont incapables d'induire la prolifération des lymphocytes T spécifiques. L'irradiation est donc un élément indispensable du procédé décrit.

### Exemple 12: la lignée de pDC pulsée et irradiée permet l'amplification massive de cellules T CD8 spécifiques d'antigènes tumoraux à partir de PBMC et de TIL de patients porteurs d'un mélanome.

La capacité des cellules GEN2.2 à induire une réponse T CD8 spécifique d'antigènes tumoraux à partir des lymphocytes T de patients porteurs d'un mélanome en situation semi-allogénique a été testée en cultivant des cellules de la lignée GEN2.2 (ci-après : cellules GEN2.2), pulsées avec un peptide d'intérêt, ou un mélange de quatre peptides d'intérêt et irradiées, avec des cellules mononuclées du sang périphériques (PBMC) ou les lymphocytes T infiltrant la tumeur (TIL) de patients HLA-A2+ porteurs d'un mélanome.

Les PBMC sont purifiées par Ficoll à partir d'un tube de sang de sujets porteurs d'un mélanome (n=12). Les pourcentages de cellules T CD8+ tétramères + spécifiques des antigènes MelanA, GP100, Tyrosinase et MAGE3 ont été mesurés initialement, ils étaient compris en moyenne entre 0.02 et 0.03%.

Les cellules tumorales et les TIL ont été purifiées à partir de biopsies tumorales, dilacérées mécaniquement puis digérées enzymatiquement (Collagénase+DNAse). Les cellules tumorales contenues dans ces suspensions cellulaires ont été séparées des TIL par leur propriété d'adhérence au plastique. Ces cellules tumorales ont été cultivées en RPMI 10% SVF et amplifiées, avant d'être congelées et utilisées comme source de cellules tumorales autologues dans certaines expériences. Les pourcentages de cellules T CD8+ tétramères + spécifiques des antigènes MelanA, GP100, Tyrosinase et MAGE3 ont été mesurés initialement dans les TIL, ils étaient en moyenne de 0,17 ; 0,2 ; 0,05 et 0,3%, respectivement.

La capacité des cellules de la lignée GEN2.2 à amplifier les lymphocytes T spécifiques d'antigènes tumoraux à partir des PBMC et TIL de patients a été évaluée.

Les cellules GEN2.2 ont été pulsées comme précédemment décrit avec un peptide d'intérêt (concentration de chaque peptide : 10µM), ou un mélange des quatre peptides issus d'antigènes tumoraux étudiés (concentration de chaque peptide : 2.5µM). Brièvement, la suspension cellulaire est incubée 3h à 37°C avec le(s) peptide(s), puis les cellules sont lavées, irradiées à 30 Gy et resuspendues à 2.10⁵ / ml en milieu RPMI complet additionné de 10% de sérum de veau foetal.

Les PBMC ou les TIL sont resuspendus à 2.10⁶ / ml en milieu RPMI complet additionné de 10% de sérum de veau foetal. Les cellules GEN2.2 pulsées et irradiées sont cocultivées avec ces lymphocytes semi-allogéniques HLA-A2+ en plaque 24 puits (2.10⁵ GEN2.2 + 2.10⁶ PBMC ou TIL / 2ml) pendant 7 jours à 37°C. Les cultures sont restimulées dans les mêmes conditions tous les 7 jours, avec ajout d'IL2 (200U/ml). Le phénotype des cellules T CD8 après 7, 14 et 20 jours de culture (J7, J14, J21). La spécificité des cellules T CD8 est analysée grâce à un marquage par des tétramères, à partir des PBMC (Figure 12a et b), et à partir des TIL (Figure 12c et d).

La coculture de PBMC avec la lignée de pDC GEN2.2 pulsée avec un peptide dérivé d'antigènes tumoraux et irradiée permet d'induire et d'amplifier des cellules T CD8 spécifiques du peptide d'intérêt avec une grande efficacité en seulement 7 jours de culture. Nous avons démontré que l'induction de telles réponses dirigées contre les antigènes tumoraux MelA, GP100, MAGE-3 ou tyrosinase était possible en utilisant cette stratégie, à partir de prélèvements de patients porteurs d'un mélanome, quel que soit le stade de leur maladie. Les PBMC des patients répondent contre au moins deux des antigènes sur les quatre étudiés, et les TIL contre au moins trois de ces antigènes.

Deux méthodes ont été utilisées pour évaluer la fonctionnalité des cellules T CD8 générées par la lignée de pDC pulsée GEN2.2 : test de cytotoxicité et sécrétion d'IFNγ après re-stimulation spécifique.

Pour évaluer la sécrétion d'IFNγ après restimulation les cellules amplifiées après culture sont récupérées, comptées et un marquage tétramère est réalisé (30 min à température ambiante). Après lavage, les cellules sont suspendues à 1.10⁶/200µl dans du milieu RPMI 10% SVF. Les cellules T2 pulsées avec le peptide d'intérêt (MelA ou GP100) ou un peptide contrôle (Flu) sont ajoutées (ratio cellules effectrices : cellules restimulantes = 10:1). Les cellules sont restimulées pendant 5h30 à 37°C en présence de brefeldine A (GolgiPlug à 1µl/ml) pendant les 3 dernières heures. Après marquage des antigènes de surface (CD3, CD8) un marquage intracellulaire d'IFNγ est ensuite réalisé (Figure 12e). L'analyse du marquage d'IFNγ est effectuée sur les cellules T CD8+ tétramère+. Ainsi des cellules T CD8+ anti-MelA générées grâce à la lignée GEN sont capables de secréter de l'IFNγ spécifiquement après re-stimulation par des cellules T2 pulsées avec MelA et pas après re-stimulation par des cellules T2 pulsées avec un autre peptide (T2 Flu), alors que des cellules T spécifiques de GP100 sécrèteront de l'IFNγ face à des T2 pulsées par GP100 mais pas par Flu.(Figure 12b).

L'activité cytotoxique des cellules T spécifiques générées par la lignée GEN2.2 est évaluée par un test de relarguage de ⁵¹Cr. Pour cela, après stimulation de PBMC de patients porteurs d'un mélanome semi-allogéniques avec des cellules GEN2.2 pulsées avec le peptide MelA et irradiées, les cellules sont récupérées et les lymphocytes T CD8+ sont purifiés (tri T CD8 EasySep par sélection négative) et mis en présence de cellules cibles marquées au ⁵¹Cr à différents ratios (E :T ratio allant de 60:1 à 7,5:1) pendant 4h. Un dosage du ⁵¹Cr dans le surnageant est ensuite effectué. Les cellules T anti-MelA sont par exemple capables de lyser efficacement des cellules tumorales HLA-A2+ qui expriment Melan-A (Me275 et Mel89) mais n'ont pas d'activité cytotoxique envers des cellules tumorales qui n'expriment pas MelA (A375) ou qui ne sont pas HLA-A2 (COLO829) (Figure 12f). Des expériences similaires ont été réalisées avec des cellules T CD8+ issues de cultures de TIL stimulées par la lignée GEN2.2 pulsée avec un mélange des quatre peptides tumoraux étudiés. L'exemple de la figure 12f (TIL patient #11) montre que les lymphocytes générés contenant des cellules tétramères+ spécifiques de MelA, GP100 et MAGE3 (Figure 12c) lysent les cellules cibles qui expriment au moins un de ces Antigènes dans le contexte HLA-A2.

La cytotoxicité des cellules T spécifiques générées par la lignée GEN2.2 pulsée par un mélange des quatre peptides tumoraux étudiés a été aussi mesurée contre les cellules tumorales autologues du patient, et des cellules non tumorales autologues (leucocytes CD45+ purifiés). Les deux exemples présentés en Figure 12g montrent pour deux patients que les TIL qui n'étaient pas cytotoxiques contre les cellules tumorales autologues initialement, acquièrent la capacité de détruire les cellules tumorales autologues après stimulation avec les cellules GEN pulsées avec le mélange des quatre peptides. Les cellules non tumorales autologues quant à elles ne sont pas lysées. Ces résultats ont été reproduits à partir de prélèvements de cinq patients (Figure 12h).

Les cellules T CD8 spécifiques d'antigènes tumoraux générées par la lignée de pDC GEN2.2 à partir de PBMC et de TIL de patients sont donc capables de tuer des cellules cibles qui expriment cet antigène de façon restreinte par le HLA, ainsi que les cellules tumorales autologues, mais pas les cellules non tumorales autologues. Elles secrètent spécifiquement de l'IFNγ après re-stimulation par des cellules présentant le peptide dont elles sont spécifiques dans le bon contexte HLA.

## Revendications

1. Méthode d'induction et d'amplification in vitro de cellules de l'immunité capables de reconnaitre au moins un antigène spécifique, comprenant les étapes suivantes:
(a) Obtention d'une lignée de cellules dendritiques plasmacytoïdes (pDCs) pulsées par incubation d'une lignée de pDC avec ledit au moins un antigène spécifique,
(b) Irradiation des cellules obtenues à l'étape (a)
(c) mise en contact de pDCs pulsées et irradiées obtenues à l'étape (b) avec des cellules mononuclées du sang périphérique (PBMC), et culture des pDC pulsées et irradiées et des PBMC partageant au moins un allèle du complexe majeur d'histocompatibilité (CMH).

2. Méthode d'induction et d'amplification selon la revendication 1 comprenant au moins une étape de re-stimulation consistant en une
(d) mise en contact des cellules obtenues à l'étape (c) à nouveau avec la lignée de pDC pulsée et irradiée obtenue à l'étape (b), et culture.

3. Méthode d'induction et d'amplification selon l'une des revendications précédentes **caractérisée en ce que** les cellules de l'immunité capables de reconnaitre au moins un antigène spécifique sont des lymphocytes T spécifiques du ou des antigène(s) utilisé(s).

4. Méthode d'induction et d'amplification selon la revendication 3 **caractérisée en ce que** les lymphocytes T sont CD8+.

5. Méthode d'induction et d'amplification selon l'une des revendications précédentes **caractérisée en ce que** le ou les antigènes sont d'origine tumorale, microbienne, bactérienne ou virale.

6. Méthode d'induction et d'amplification selon la revendication 5 **caractérisée en ce que** le au moins un antigène est un peptide.

7. Méthode d'induction et d'amplification selon la revendication 6 **caractérisée en ce que** le peptide, susceptible d'être obtenu à partir d'un antigène d'origine tumorale, est choisi parmi les peptides compris dans la séquence des antigènes CEA, NY-BR1 , Her-2/Neu, PSA, RAGE-1 , PRAME, TRP-2, MAGE-A1 , MAGE-A2, MAGE-A4, MAGE-A9, MAGE-A10, MAGE-C2, MUC-1, p53, hTERT, survivin, melan-A/MART-1 , GP100, tyrosinase, MAGE-A3 ou NY-ESO1.

8. Méthode d'induction et d'amplification selon la revendication 5 **caractérisée en ce que** le peptide, susceptible d'être obtenu à partir d'un antigène d'origine virale, est choisi parmi les peptides compris dans la séquence des antigènes env, nef, gp41 , gp120, gag ou pol du virus HIV, HBc ou HBs du virus HBV, core, NS3 ou NS5 du virus HCV, Flu M 1 du virus influenza, pp65 du virus CMV, BMLF1 , LMP2, EBNA-2 ou EBNA-3a du virus EBV.

9. Méthode d'induction et d'amplification selon l'une des revendications précédentes **caractérisée en ce que** la lignée de pDC est obtenue à partir de cellules de leucémie à pDC.

10. Méthode d'induction et d'amplification selon la revendication 9 **caractérisée en ce que** la lignée de pDC est la lignée GEN 2.2 ou la lignée GEN3.

11. Utilisation d'une lignée de pDC incubée avec au moins un antigène d'origine tumorale, microbienne, bactérienne ou virale et irradiée pour la fabrication d'un médicament pour la prévention et/ou le traitement de maladies infectieuses ou de cancers.

12. Composition comprenant une lignée de pDC pulsées par incubation avec un antigène tumoral ou un antigène viral, ledit antigène viral étant choisi parmi les peptides compris dans la séquence des antigènes env, nef, gp41 , gp120, gag ou pol du virus HIV, HBc ou HBs du virus HBV, core, NS3 ou NS5 du virus HCV, flu M1 du virus influenza, pp65 du virus CMV, BMLF1 , LMP2, EBNA-2 ou EBNA-3a du virus EBV, et irradiées.

13. Composition comprenant une culture de cellules, ladite culture comprenant une lignée de pDC incubée avec au moins un antigène et irradiée et des cellules mononuclées du sang périphérique (PBMC), les pDC incubées avec au moins un antigène et irradiées et les PBMC partageant au moins un allèle du complexe majeur d'histocompatibilité (CMH).

14. Composition pharmaceutique comprenant une lignée de pDC incubée avec au moins un antigène et irradiées pour son utilisation comme médicament.

15. Lignée de pDC incubée avec au moins un antigène et irradiée pour son utilisation vaccinale comme agent d'activation du système immunitaire.

16. Kit comprenant une lignée de pDC incubée avec au moins un antigène et irradiée et des cellules mononuclées du sang périphérique (PBMC), la lignée de pDC et les PBMC partageant au moins un allèle du complexe majeur d'histocompatibilité (CMH).

## Patentansprüche

1. Verfahren zur in vitro Induktion und Amplifikation von Immunitätszellen, die in der Lage sind, mindestens ein spezifisches Antigen wiederzuerkennen, umfassend die folgenden Schritte:
(a) Gewinnung einer gepulsten, plasmazytoiden dendritischen Zelllinie (pDCs) durch Inkubieren einer pDC-Linie mit mindestens einem spezifischen Antigen,
(b) Bestrahlung der in Schritt (a) erhaltenen Zellen,
(c) In-Kontakt-bringen der in Schritt (b) erhaltenen gepulsten und bestrahlten pDCs mit mononukleären Zellen des peripheren Blues (PBMC) und Kultivieren der gepulsten und bestrahlten pDCs und der PBMC, die mindestens ein Allel des Haupthistokompatibilitätskomplexes (CMH) teilen.

2. Verfahren zur Induktion und Amplifikation gemäß Anspruch 1, umfassend mindestens einen Schritt der Restimulation, bestehend aus einem
(d) erneuten In-Kontakt-bringen der in Schritt (c) erhaltenen Zellen mit der in Schritt (b) erhaltenen, gepulsten und bestrahlten pDC-Linie und Kultivieren.

3. Verfahren zur Induktion und Amplifikation gemäß einem der voranstehenden Ansprüche, **dadurch gekennzeichnet, dass** die Immunitätszellen, die in der Lage sind, mindestens ein spezifisches Antigen wiederzuerkennen, für den oder die verwendeten Antikörper spezifische T-Lymphozyten sind.

4. Verfahren zur Induktion und Amplifikation gemäß Anspruch 3, **dadurch gekennzeichnet, dass** die T-Lymphozyten CD8+ sind.

5. Verfahren zur Induktion und Amplifikation gemäß einem der voranstehenden Ansprüche, **dadurch gekennzeichnet, dass** das oder die Antigene tumorösen, mikrobiellen, bakteriellen oder viralen Ursprungs sind.

6. Verfahren zur Induktion und Amplifikation gemäß Anspruch 5, **dadurch gekennzeichnet, dass** mindestens ein Antigen ein Peptid ist.

7. Verfahren zur Induktion und Amplifikation gemäß Anspruch 6, **dadurch gekennzeichnet, dass** das Peptid, erhältlich ausgehend von einem Antigen tumorösen Ursprungs, ausgewählt ist aus den Peptiden umfasst in der Sequenz der Antigene CEA, NY-BR1 , Her-2/Neu, PSA, RAGE-1 , PRAME, TRP-2, MAGE-A1 , MAGE-A2, MAGE-A4, MAGE-A9, MAGE-A10, MAGE-C2, MUC-1 ,p53, hTERT, Survivin, Melan-A/MART-1, GP100, Tyrosinase, MAGE-A3 oder NY-ESO1.

8. Verfahren zur Induktion und Amplifikation gemäß Anspruch 5, **dadurch gekennzeichnet, dass** das Peptid, erhältlich ausgehend von einem Antigen viralen Ursprungs, ausgewählt ist aus den Peptiden umfasst in der Sequenz der Antigene Env, Nef, gp41 , gp120, Gag oder Pol des HIV-Virus, HBc oder HBs des HBV-Vvirus, Core, NS3 oder NS5 des HCV-Virus, FIu M1 des Influenza-Virus, pp65 des CMV-Virus, BMLF1 , LMP2, EBNA-2 oder EBNA-3a des EBV-Virus.

9. Verfahren zur Induktion und Amplifikation gemäß einem der voranstehenden Ansprüche, **dadurch gekennzeichnet, dass** die pDC-Linie erhalten wird ausgehend von leukämischen Zellen zum pDC.

10. Verfahren zur Induktion und Amplifikation gemäß Anspruch 9, **dadurch gekennzeichnet, dass** die pDC-Linie die Linie GEN 2.2 oder die Linie GEN3 ist.

11. Verwendung einer mit mindestens einem Antigen tumorösen, mikrobiellen, bakteriellen oder viralen Ursprungs inkubierten und bestrahlt pDC-Linie für die Herstellung eines Medikamentes für die Prävention und/oder die Behandlung von infektiösen Krankheiten oder Krebs.

12. Zusammensetzung umfassend eine durch Inkubation mit einem tumorösen Antigen oder einem viralen Antigen gepulste, wobei das virale Antigen ausgewählt ist aus den Peptiden umfasst in der Sequenz der Antigene Env, Nef, gp41 , gp120, Gag oder Pol des HIV-Virus, HBc oder HBs des HBV-Vvirus, Core, NS3 oder NS5 des HCV-Virus, Flu MI des Influenza-Virus, pp65 des CMV-Virus, BMLF1 , LMP2, EBNA-2 oder EBNA-3a des EBV-Virus, und bestrahlte pDC-Linie.

13. Zusammensetzung umfassend eine Zellkultur, wobei die Kultur eine mit mindestens einem Antigen inkubierte und bestrahlte pDC-Linie und mononukleäre Zellen des peripheren Blutes (PBMC) umfasst, wobei die mit mindestens einem Antigen inkubierten und bestrahlten pDC und die PBMC mindestens ein Allel des Haupthistokompatibilitätskomplexes (CMH) teilen.

14. Pharmazeutische Zusammensetzung umfassend eine mit mindestens einem Antigen inkubierte und bestrahlte pDC-Linie, für ihre Verwendung als Medikament.

15. Mit mindestens einem Antigen inkubierte und bestrahlte pDC-Linie für ihre Impfbezogene Verwendung als Mittel zur Aktivierung des Immunsystems.

16. Kit umfassend eine mit mindestens einem Antigen inkubierte und bestrahlte pDC-Linie und mononukleäre Zellen des peripheren Blutes (PBMC), wobei die pDC-Linie und die PBMC mindestens ein Allel des Haupthistokompatibilitätskomplexes (CMH) teilen.

## Claims

1. A method for *in vitro* inducing and amplifying immune cells capable of recognizing at least one specific antigen, comprising the following steps:
(a) obtaining a plasmacytoid dendritic (pDC) cell line pulsed by incubation of a pDC line with said at least one specific antigen,
(b) irradiating the cells obtained at step (a)
(c) bringing pulsed and irradiated pDCs obtained at step (b) into contact with peripheral blood mono-nuclear cells (PBMC), and culturing the pulsed and irradiated pDCs and the PBMC sharing at least one major histocompatibility complex (MHC) allele.

2. The method for inducing and amplifying according to claim 1, comprising at least one restimulation step consisting in
(d) bringing the cells obtained at step (c) into contact again with the pulsed and irradiated pDC line obtained at step (b), and culturing.

3. The method for inducing and amplifying according to one of the preceding claims **characterized in that** the immune cells capable of recognizing at least one specific antigen are T lymphocytes specific for the antigen(s) used.

4. The method for inducing and amplifying according to claim 3 **characterized in that** the T lymphocytes are CD8+.

5. The method for inducing and amplifying according to one of the preceding claims **characterized in that** the antigen(s) is (are) of tumor, microbial, bacterial or viral origin.

6. The method for inducing and amplifying according to claim 5 **characterized in that** the at least one antigen is a peptide.

7. The method for inducing and amplifying according to claim 6 **characterized in that** the peptide, which can be obtained from an antigen of tumor origin, is chosen from the peptides included in the sequence of the antigens CEA, NY-BR1, Her-2/Neu, PSA, RAGE-1, PRAME, TRP-2, MAGE-A1, MAGE-A2, MAGE-A4, MAGE-A9, MAGE-A10, MAGE-C2, MUC-1, p53, hTERT, survivin, melan-A/MART-1, GP100, tyrosinase, MAGE-A3 or NY-ESO1.

8. The method for inducing and amplifying according to claim 5 **characterized in that** the peptide, which can be obtained from an antigen of viral origin, is chosen from the peptides included in the sequence of the antigens env, nef, gp41, gp120, gag or pol of the HIV virus, HBc or HBs of the HBV virus, core, NS3 or NS5 of the HCV virus, Flu M1 of the influenza virus, pp65 of the CMV virus, BMLF1, LMP2, EBNA-2 or EBNA-3a of the EBV virus.

9. The method for inducing and amplifying according to one of the preceding claims **characterized in that** the pDC line is obtained from pDC leukemia cells.

10. The method for inducing and amplifying according to claim 9 **characterized in that** the pDC line is the GEN2.2 line or the GEN3 line.

11. Use of a pDC line incubated with at least one antigen of tumor, microbial, bacterial or viral origin and irradiated for producing a medicament for the prevention and/or the treatment of infectious diseases or cancers.

12. A composition comprising a pDC line pulsed by incubation with a tumor antigen or a viral antigen, said viral antigen being chosen from the peptides included in the sequence of the antigens env, nef, gp41, gp120, gag or pol of the HIV virus, HBc or HBs of the HBV virus, core, NS3 or NS5 of the HCV virus, flu M1 of the influenza virus, pp65 of the CMV virus, BMLF1, LMP2, EBNA-2 or EBNA-3a of the EBV virus, and irradiated.

13. A composition comprising a culture of cells, said culture comprising a pDC line incubated with at least one antigen and irradiated and peripheral blood mononuclear cells (PBMC), the pDC incubated with at least one antigen and irradiated and the PBMC sharing at least one major histocompatibility complex (MHC) allele.

14. A pharmaceutical composition comprising a pDC line incubated with at least one antigen and irradiated for use as a medicament.

15. A pDC line incubated with at least one antigen and irradiated for vaccinal use as an activating agent of the immune system.

16. A kit comprising a pDC line incubated with at least one antigen and irradiated and peripheral blood mononuclear cells (PBMC), the pDC line and the PBMC sharing at least one major histocompatibility complex (MHC) allele.
